# DEMANDE DE BREVET EUROPEEN

(11) **EP 4 248 855 A1**
(43) Date de publication de la demande: **27.09.2023**
(21) Numéro de dépôt: 22214368.7
(22) Date de dépôt: 16.12.2022
(51) Int. Cl.: A61B 5/0535, A61B 5/282, A61B 5/308, G01G 19/50, A61B 5/305, A61B 5/11, A61B 5/00

(54) **STATION DE MESURE AVEC MESURE D'ELECTROCARDIOGRAMME**

(30) Priorité: 31.12.2021 FR 2114744
(71) Demandeur: Withings, 92130 Issy-les-Moulineaux (FR)
(72) Inventeur: Wang, Jean-Louis, 92130 Issy-les-Moulineaux (FR); Merlot, Antoine, 92130 Issy-les-Moulineaux (FR)
(74) Mandataire: Withings IP

(57) **Abrégé**

La présente description concerne une station de mesure comprenant :
- un système d'acquisition d'électrocardiogramme, dit ECG, (1010),
- deux électrodes de contrôle (1022, 1024) configurées pour être au contact d'un utilisateur,
- un circuit de connexion électrique (1026), le circuit de connexion électrique comprenant une boucle de rétroaction (1028) connectée aux deux électrodes de contrôle (1022, 1024).

## Description

### Domaine technique

La présente description concerne la surveillance de la santé d'un utilisateur et plus spécifiquement concerne les stations de mesure permettant de mettre en oeuvre une ou plusieurs mesures de signaux biométriques (ou paramètres physiologiques) d'un utilisateur.

Au moins une des données suivantes est déterminée par la station de mesure de la présente demande : poids ou masse, électrocardiogramme (ECG), impédancemétrie (analyse d'impédance du corps humain), dont impédance-pléthysmogramme (IPG) impedance-cardiogramme (ICG) et bioimpédance (« *bioimpedance analysis* » BIA, pour la masse de graisse, d'eau, de muscles, etc.), photoplethysmogramme (PPG), ballistocardiogramme (BCG), analyse de la conductance électrochimique de la peau (« ESC analysis » pour « *electrochemical skin conductance* » ou plus simplement « ESC » dans la présente description) et évaluation de la fonction sudorale (parfois appelée « *sudogramme* » dans la présente demande), rythme cardiaque (« *heart rate* », HR), vitesse d'onde de pouls vitesse d'onde de pouls (« *pulse wave velocity* », PWV), etc.

### Etat de la technique

Le document WO2010/122252 décrit une balance connectée avec mesure de poids et de bioimpédance. Les documents EP3087914 et EP3095380 décrivent quant à eux une balance connectée permettant d'obtenir des informations sur l'état cardiovasculaire de l'utilisateur, avec notamment une mesure de PTT (« pulse transit time ») à l'aide d'un BCG et d'un IPG. Le document WO2021/164561 décrit une balance avec poignée permettant de mesurer un poids, d'effectuer un ECG multi-voies et d'effectuer une BIA segmentale.

Pour obtenir un ECG de qualité, une technique consiste à utiliser une électrode dite « de jambe droite », appelée RLD en anglais (« right leg drive »), qui permet de mesurer un potentiel du corps d'un utilisateur pour réinjecter une version amplifiée et inversée de ce potentiel.

Le document WO2020/081471 décrit un système avec un ensemble d'électrodes permettant d'effectuer un ECG et une électrode RLD. Alternativement, ce document propose de ne pas utiliser de RLD et d'utiliser un signal couplé alternatif décentré de la moitié de l'alimentation (« a signal input is AC-coupled and biased at mid-supply voltage »).

En particulier, l'obtention d'un IPG et d'un ECG synchronisés est recherché pour différentes raisons : meilleure identification des pics, meilleure connaissance de l'état vasculaire de l'utilisateur, etc.

Le document « Estimation of Puise Arrival Time Using Impedance Plethysmogram from Body Composition Scale" de Paliakatie et al. décrit un ECG et un IPG synchronisé. L'ECG et l'IPG sont acquis par deux systèmes différents mais la synchronisation n'est pas décrite (la transmission sans-fil est généralement source de difficulté).

Le document « Pulse arrival Time Estimation from the Impédance Plethysmogram Obtained with a Handheld device" de Gomez-Clapers et al. décrit un système avec quatre électrodes au contact des mains avec une masse flottante *(« amplifier common »)* reliée directement aux électrodes pour obtenir un ECG-1-voie (entre les bras) et un IPG synchronisés. Le système est localisé et est donc moins bruité, comme expliqué dans la référence [15] (*"*Fast and Easy-to-Use ECG Acquisition and Heart Rate Monitoring System Using a Wireless Steering Wheel de ce document", Gomez-Clapers).

Le document EP2737847 décrit une méthode comprenant une mesure d'ECG et d'IPG sur les jambes, à l'aide de deux circuits indépendants. Le document ne rentre pas davantage dans le détail.

### Présentation de l'invention

Effectuer un ECG et un IPG en même temps génère des difficultés, à plus forte raison lorsque les deux mesures sont effectuées par le même dispositif. Lorsque l'ECG est un ECG multivoie avec des électrodes sur le bras et les jambes de l'utilisateur (par exemple via une poignée et une balance), la présence de bruit est généralement plus importante.

La présente description vise à proposer une station de mesure permettant l'obtention de différentes mesures de signaux biométriques, avec une qualité accrue.

L'invention est définie dans les revendications.

Dans un mode de réalisation, la description présente une station de mesure comprenant :
- un système d'acquisition d'électrocardiogramme, dit système d'acquisition ECG,
- deux électrodes de contrôle configurées pour être au contact d'un utilisateur,
- un circuit de connexion électrique, le circuit de connexion électrique comprenant une boucle de rétroaction connectée aux deux électrodes de contrôle.

Dans un mode de réalisation, l'une des deux électrodes de contrôle est configurée pour mesurer un potentiel du corps, la boucle de rétroaction est configurée pour amplifier et inverser le potentiel mesuré, et l'autre des deux électrodes de contrôle est configurée pour mettre le corps au potentiel amplifié et inversé.

En particulier, le système d'acquisition ECG comprend une pluralité d'électrodes ECG, configurées pour être au contact d'un utilisateur et un circuit électronique ECG, connecté à la pluralité d'électrodes ECG. La pluralité d'électrode ECG peut inclure au moins une électrode sur le pied et au moins une électrode sur les mains ou bien deux électrodes sur les deux pieds ou bien deux électrodes sur les deux mains. Dans un exemple, le système d'acquisition ECG comprend trois électrodes ECG, deux sur les mains et une sur le pied gauche.

Dans un mode de réalisation, les deux électrodes de contrôle sont distinctes de la pluralité d'électrodes ECG. Dans un mode de réalisation, le circuit de connexion électrique est électriquement indépendant du circuit électronique ECG.

Dans un mode de réalisation, la boucle de rétroaction comprend :
- un amplificateur opérationnel, et
- une connexion de rétroaction, reliant une sortie de l'amplificateur opérationnel à une entrée de l'amplificateur opérationnel, la connexion de rétroaction comprenant au moins un composant passif.

Dans un mode de réalisation, le composant passif comprend une résistance et/ou un condensateur.

Dans un mode de réalisation, les électrodes de contrôle sont reliées respectivement à l'entrée et à la sortie de l'amplificateur opérationnel.

Dans un mode de réalisation, la station de mesure comprend un système d'impédancemétrie, le système d'impédancemétrie comprenant :
- une source de courant, par exemple de courant alternatif,
- deux électrodes d'injection de courant, pour injecter un courant dans le corps d'un utilisateur,
la source de courant étant reliée au circuit de connexion électrique et les deux électrodes d'injection de courant étant les deux électrodes de contrôle.

Dans un mode de réalisation, la station de mesure comprend un commutateur permettant de déconnecter le circuit de connexion électrique et la source de courant ou dans laquelle la source de courant est désactivable.

Dans un mode de réalisation, le système d'impédancemétrie comprend deux électrodes de mesure, aptes à mesurer une différence de potentiel d'une portion du corps de l'utilisateur traversée par un courant généré par la source de courant.

Dans un mode de réalisation, le système d'acquisition d'ECG et le système d'impédancemétrie sont configurés pour être activés simultanément, afin d'acquérir un ECG et une mesure d'impédance synchronisés, par exemple un impédance-pléthysmogramme, IPG.

Dans un mode de réalisation, la station de mesure comprend une base apte à recevoir les pieds d'un utilisateur et sur laquelle est montée au moins une électrode de contrôle.

Dans un mode de réalisation, la station de mesure comprend une poignée, apte à recevoir les mains d'un utilisateur, et sur laquelle est montée au moins une électrode parmi les électrodes ECG.

Dans un mode de réalisation, les électrodes de contrôle sont montées sur la base, deux électrodes ECG sont montées sur la poignée, et une électrode ECG est montée sur la base.

La description présente aussi un procédé d'acquisition d'un électrocardiogramme, ECG, à l'aide d'une station de mesure telle que décrite précédemment, comprenant les étapes suivantes :
- E1 : connexion des électrodes de contrôle au circuit de connexion électrique, par un commutateur,
- E3 : activation du système d'acquisition ECG pour acquérir un ECG.

Dans un mode de réalisation, l'étape suivante est mise en oeuvre avant l'étape E3 d'activation du système d'acquisition ECG :
- E2 : désactivation de la source de courant ou déconnexion de la source de courant du circuit de connexion électrique.

La description présente aussi un procédé d'acquisition d'un électrocardiogramme, ECG, et d'une mesure d'impédance, par exemple un IPG, à l'aide d'une station de mesure telle que décrite précédemment, comprenant les étapes suivantes :
- F1 : connexion des électrodes de contrôle au circuit de connexion électrique, par le commutateur,
- F3 : activation simultanée du système d'acquisition ECG et du système d'impédancemétrie pour acquérir un ECG et une mesure d'impédance.

Dans un mode de réalisation, la description présente aussi une station de mesure comprenant :
- un système d'impédancemétrie comprenant :
   ∘ au moins deux électrodes d'injection, pour injecter un courant dans un utilisateur, et
   ∘ un circuit de connexion électrique, le circuit de connexion électrique comprenant une boucle de rétroaction connectée aux deux électrodes d'injection,
- un système d'acquisition d'électrocardiogramme, ECG, configuré pour acquérir des signaux d'ECG,
- une première électrode de contrôle, configurée pour modifier le potentiel du corps de l'utilisateur, lequel la première électrode de contrôle étant l'une des électrodes d'injection de courant.

Dans un mode de réalisation, la station de mesure comprend une deuxième électrode de contrôle, la deuxième électrode de contrôle étant l'autre des deux électrodes d'injection de courant.

Dans un mode de réalisation, le système d'acquisition ECG comprend :
∘ une pluralité d'électrodes ECG, configurées pour être au contact d'un utilisateur,
∘ un circuit électronique ECG, connecté à la pluralité d'électrodes ECG, et
dans laquelle une des électrode de contrôle est une des électrodes ECG.

Dans un mode de réalisation, la description présente une station de mesure comprenant :
∘ une base configurée pour recevoir les pieds d'un utilisateur et comprenant au moins une électrode de pied apte à être en contact avec un pied d'un utilisateur,
∘ une poignée configurée pour recevoir les mains d'un utilisateur et comprenant au moins une électrode de main,
∘ un système de détection pour détecter un contact simultané de l'utilisateur avec l'électrode de pied et avec l'électrode de main.

Le système de détection peut générer une donnée indiquant que la détection a eu lieu et une donnée que la détection n'a pas eu lieu ou tout simplement ne pas générer de signal.

Dans un mode de réalisation, le système de détection est configuré pour mettre une électrode des deux électrodes à un potentiel et pour détecter par l'autre électrode un potentiel supérieur à un seuil.

Dans un mode de réalisation, le système de détection comprend :
∘ un générateur de tension apte à mettre ladite électrode au potentiel et
∘ un voltmètre apte à mesure le potentiel de ladite électrode.

Dans un mode de réalisation, la station de mesure comprend système d'acquisition de mesure (BIA, ECG, IPG) utilisant les deux électrodes et la station de mesure est configurée pour déclencher le système d'acquisition de mesure en cas de détection d'un contact simultané par le système de détection.

Dans un mode de réalisation, la station de mesure comprend une circuiterie de contrôle configurée pour mettre en oeuvre une séquence prédéterminée de mesures à exécuter par un ou plusieurs systèmes d'acquisition de mesure (BIA, ECG, IPG), les mesures nécessitant la poignée n'étant pas exécutées en l'absence de détection d'un contact simultané par le système de détection.

La circuiterie de contrôle peut passer d'une mesure à l'autre, sans nécessiter de mesure au moyen de la poignée.

Dans un mode de réalisation, la station de mesure comprend une circuiterie de contrôle configurée pour mettre en oeuvre une séquence prédéterminée de mesures à exécuter par un ou plusieurs systèmes d'acquisition de mesure (BIA, ECG, IPG), les mesures nécessitant la poignée étant exécutées en cas de détection d'un contact simultané par le système de détection.

Dans un mode de réalisation, le système d'acquisition de mesure comprend un système d'acquisition d'ECG ou un système d'impédancemétrie.

Dans un mode de réalisation, en réponse à une absence de détection d'un contact simultané par le système de détection, le système de d'acquisition d'ECG n'est pas activé.

Dans un mode de réalisation, la station de mesure comprend une circuiterie de contrôle apte à mettre en oeuvre les étapes suivantes :
∘ imposition d'un potentiel à une électrode (dans le pied par exemple)
∘ mesure du potentiel au niveau de l'autre électrode,
∘ détermination que le potentiel mesuré est au moins égal à un seuil prédéterminé, le seuil dépendant du potentiel imposé (le seuil étant par exemple égal à la valeur du potentiel imposé moins une valeur de perte du corps de l'utilisateur).

Dans un mode de réalisation, en réponse à la détection d'un contact simultané par le système de détection, la circuiterie de contrôle permet une acquisition de mesure utilisant les électrodes de la poignée.

Dans un mode de réalisation, le potentiel est compris entre 0,5V et 2V.

Dans un mode de réalisation, la base comprend un système de mesure de poids incluant un capteur de poids et la station de mesure est configurée pour activer le système de détection en réponse à une détection de poids par le système de mesure de poids.

Dans un mode de réalisation, la station de mesure comprend une circuiterie de contrôle stockant une séquence de mesures comprenant des mesures avec poignée et des mesures sans poignée et une séquence de mesure comprenant uniquement des mesures sans poignée, dans laquelle la circuiterie de contrôle est configurée pour basculer d'une séquence à l'autre en fonction de la détection par le système de détection. En particulier, si le système de détection détecte le contact simultané, alors la circuiterie de contrôle choisie la séquence complète de mesures.

La présente description présente aussi un procédé permettant de détecter un contact entre l'utilisateur et les électrodes, à l'aide de la station de mesure décrite précédemment.

### Description des figures

Les figures suivantes illustrent les éléments décrits dans la présente description.
FIG. 1 : la figure 1 représente une vue tridimensionnelle d'une station de mesure avec une poignée, selon un mode de réalisation ;
FIG. 2 : la figure 2 représente une vue latérale de la station de la figure 1 ;
FIG. 3 : la figure 3 représente une vue tridimensionnelle de la station de la figure 1, mais avec la poignée en position déployée ;
FIG. 4 : la figure 4 représente une vue détaillée de la poignée ;
FIG. 5 : la figure 5 représente une vue schématique de la station de mesure et de son environnement ;
FIG. 6 : la figure 6 illustre une vue tridimensionnelle d'une plaque de mesure isolée ;
FIG. 7 : la figure 7 illustre une vue du dessous la plaque de mesure de la figure 6 ;
FIG. 8 : la figure 8 illustre une vue d'un système d'acquisition d'ECG avec une électrode RLD ;
FIG. 9 : la figure 9 illustre des mesures d'un ECG avec une électrode RLD ;
FIG. 10 : la figure 10 illustre une vue schématique d'un système d'acquisition d'ECG avec un dispositif de rétroaction selon un mode de réalisation de l'invention ;
FIG. 11 : la figure 11 illustre une vue schématique d'un système d'acquisition d'ECG avec un dispositif de rétroaction qui est intégré à un système d'impédancemétrie, selon un mode de réalisation de l'invention ;
FIG. 12 : la figure 12 illustre des mesures obtenues avec le schéma de la figure 11, sans IPG,
FIG. 13 : la figure 13 illustre des mesures obtenues avec le schéma de la figure 11, avec IPG,
FIG. 14 : la figure 14 illustre un procédé d'acquisition d'ECG,
FIG 15 : la figure 15 illustre un procédé d'acquisition synchronisée d'ECG et d'IPG.

### Description détaillée

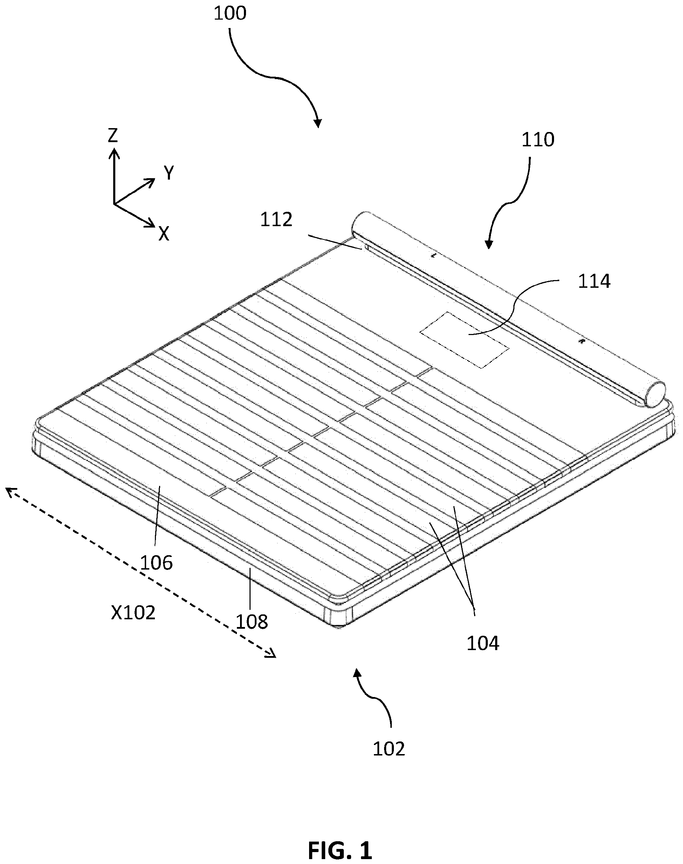

Les figures 1 à 4 illustrent une représentation d'une station de mesure 100 selon au moins un mode de réalisation de la présente description. La station de mesure 100 se présente essentiellement sous la forme d'une base 102 sur laquelle un utilisateur peut poser ses pieds, par exemple à plat. L'utilisateur peut être sur la station de mesure ou assis sur une chaise. En position normale d'utilisation, les pieds de l'utilisateur sont posés à plat sur la station de mesure 100.L'épaisseur de la base 102 est par exemple inférieure à 10 cm, voire 6cm. La station de mesure 100 comprend un ou plusieurs capteurs 104 aptes à mesurer des informations physiologiques d'un utilisateur.

Dans un mode de réalisation, certains capteurs 104 (par exemple des électrodes) sont montés sur un substrat 106 de la base 102, le substrat étant configuré pour recevoir les pieds d'un utilisateur. Le substrat peut être une plaque rigide, comme illustré sur les figures, et appelée plaque de mesure 106. La plaque de mesure 106 définit un plan parallèle à un plan XY. La plaque de mesure 106 peut être en verre. Néanmoins, le substrat peut être déformable sous le poids de l'utilisateur. Le substrat 106 peut être monté sur un socle 108, par exemple rigide, ou des pieds (non illustré). Dans le cas d'une base 102 fonctionnant comme un pèse-personne, des capteurs sont positionnés entre le substrat 106 et le socle 108 (architecture dite « sandwich ») ou entre le substrat 106 et les pieds (architecture dite « à pieds »). Les capteurs peuvent être des cellules de charge (généralement quatre) qui permettent d'obtenir un poids, et donc une masse d'un utilisateur. Le socle 108 peut être en métal ou en plastique.

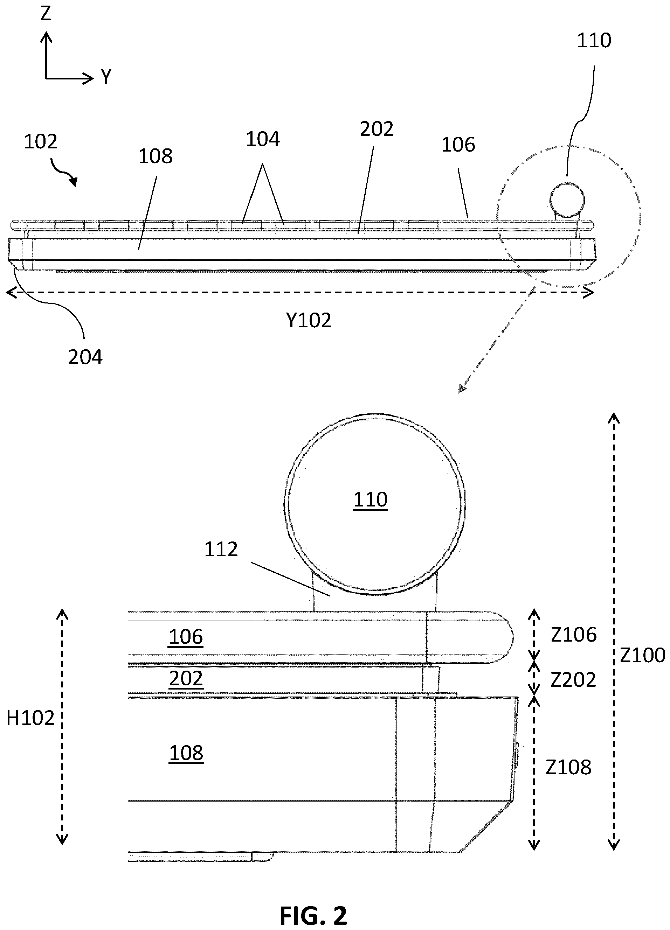

Comme visible en figure 2, la station de mesure 100 comprend une plaque de support 202, qui peut être solidaire de la plaque de mesure 106. La plaque de support 202 est conçue pour recevoir une partie de l'électronique de la station de mesure 100. La plaque de support 202 est positionnée entre le socle 108 et la plaque de mesure 106.

Dans une architecture à pieds, on définit deux groupes structurels en déplacement l'un par rapport à l'autre : les pieds d'une part (groupe fixe), et tout le reste d'autre part (groupe mobile). Les cellules de charges relient mécaniquement ces deux groupes. La plaque de support, si présente, est alors généralement cachée par un capot externe solidaire de la plaque de mesure. Visuellement, seule la partie mobile est généralement visible.

Dans une architecture sandwich, on définit deux groupes en déplacement l'un par rapport à l'autre : le socle 108 et les éléments associés à ce dernier d'une part (groupe fixe), et tout le reste d'autre part (groupe mobile). Les cellules de charges relient mécaniquement ces deux groupes. Visuellement, les deux groupes sont généralement visibles.

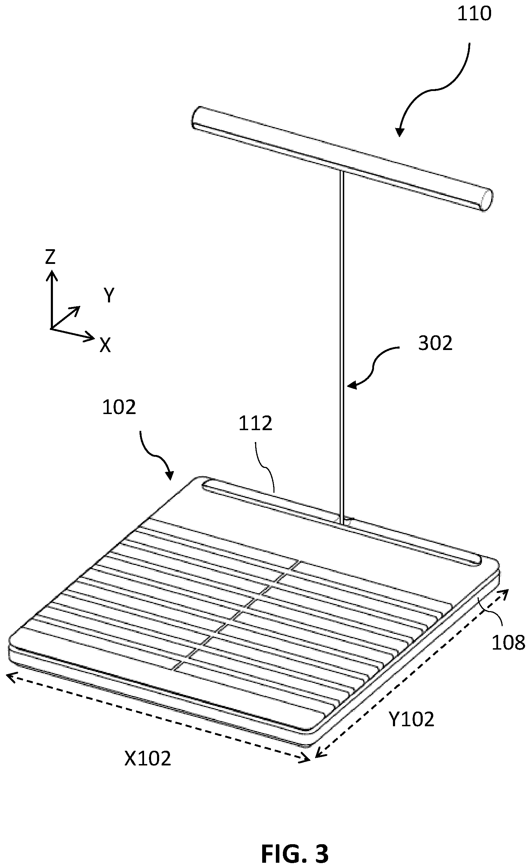

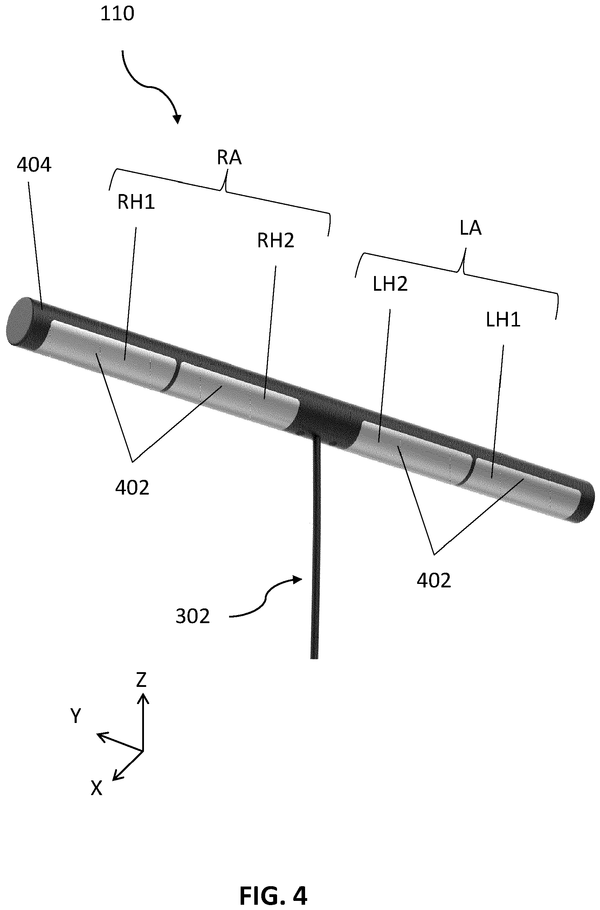

Dans un mode de réalisation, la station de mesure 100 comprend en outre une poignée 110, apte à être saisie par une moins une main de l'utilisateur, illustrée en figures 3 et 4. La poignée 110 peut être reliée à la station de mesure par un câble 302 (visible sur la figure 3). Afin d'avoir une station de mesure 100 pratique et sans câble volant, le câble 302 peut se déployer et se rétracter (par exemple s'enrouler et se dérouler) à l'intérieur de la base 102. A cette fin, un enrouleur (non visible les figures) est agencé dans un espace prévu entre le substrat 106 et le socle 108. Au moins deux positions sont ainsi définies pour la poignée : une position rangée (visible en figures 1 et 2) et une position déployée (visible en figure 3). La base 102 comprend en outre un support de poignée 112 qui peut accueillir la poignée 110 en position rangée. Le support de poignée 112 est par exemple monté sur le substrat 106. Il sera décrit plus en détail par la suite. La poignée 110 comprend elle aussi au moins un capteur 104, qui est référencé 402.

La poignée 110 est utilisée pour effectuer au moins une mesure parmi les mesures suivantes : ECG (ECG-1-voie entre les deux mains ou plusieurs voies avec d'autres membres), BIA (dites « segmentales »), IPG. Les capteurs 402 de la poignée 110 sont choisis notamment parmi : capteur optique pour PPG et électrodes.

La base 102 peut comprendre un affichage 114 (par exemple un écran ou un affichage à LED ou à encre électronique) pour afficher des informations à destination de l'utilisateur. L'écran 114 est affiché en pointillé sur la figure 1 car, dans l'exemple des figures, il est situé sous la plaque de mesure 106 et n'est pas ou peu visible lorsqu'il est éteint.

Le socle 108 de la base 102 peut comprendre un chanfrein 204 pour faciliter la préhension de la station de mesure 100 lorsqu'elle est sur le sol.

Dans un mode de réalisation, la base 102 a une forme essentiellement rectangulaire dans un plan XY. La base 102 a par exemple une forme essentiellement parallélépipédique dans l'espace XYZ.

Lorsque la station de mesure 100 est positionnée à plat, la plaque de mesure 106 est parallèle à un plan XY. La station de mesure 100 comprend une direction longitudinale dans un plan XY et une dimension transversale dans un plan XY et orthogonale à la direction longitudinale. Par hauteur, on entend la dimension selon l'axe Z (aussi appelé épaisseur) ; par largeur, il est signifié la dimension transversale selon l'axe X ; par longueur, il est signifié la dimension longitudinale selon l'axe Y. En utilisation normale, les pieds de l'utilisateur se positionnent le long de la longueur Y de la base 102. Le bord de la station de mesure 110 (ou de la base 102, ou bien encore de la plaque de mesure 106) qui est le plus proche de la partie antérieure du pied en utilisation normale (c'est à dire les doigts de pied) est appelé bord antérieur et le bord opposé de la station de mesure 110 (ou de la base 102, ou bien encore de la plaque de mesure 106), qui est le plus proche de la partie postérieure du pied en utilisation normale (c'est-à-dire le talon) est appelé bord postérieur. On peut définir un axe médian, selon la longueur Y (longitudinale donc), autour duquel la plaque de mesure 106 est symétrique et qui permet de définir une partie gauche, destinée au pied gauche, et une partie droite, destinée au pied droit. La largeur X102 de la base 102 peut être comprise entre 330 et 400mm (par exemple environ 357mm) et la longueur de la base Y102 peut être comprise entre 300 et 360mm (par exemple environ 325mm). La longueur et/ou la largeur de la plaque de mesure 106 peuvent être légèrement moindre que celles du socle 108, de sorte que la plaque de mesure 106 soit légèrement en retrait par rapport au socle 108. Dans ce cas, la longueur et la largeur du socle 108 correspondent respectivement à la longueur et à la largeur données ci-dessus pour la base 102. Une telle conception permet de protéger la plaque de mesure 106 des chocs et des contacts avec l'environnement extérieur. La demande FR2106653, décrit une telle solution.

Concrètement, comme illustré en figure 2 : la hauteur Z102 de la base 102 peut être comprise entre 20 et 35mm (par exemple entre 35 et 40mm) et la hauteur maximale Z100 de la station de mesure 100 peut être comprise entre 45 et 55mm (par exemple 51mm). Comme illustré sur les figures, seuls le support de poignée 112 et la poignée 110 sont en saillie selon la direction Z par rapport à la plaque de mesure 106. En détaillant les différentes dimensions : la hauteur Z108 du socle 108 peut être comprise entre 10 et 20mm (par exemple 18mm), la hauteur Z202 de la plaque de support 202 peut être comprise entre 3 et 6mm (par exemple 4mm), la hauteur Z106 de la plaque de mesure 106 peut être comprise entre 4 et 8mm (par exemple 6mm).

Le câble 302 peut avoir une longueur comprise entre 50cm et 120cm. La longueur est choisie pour que la plupart des utilisateurs puisse saisir la poignée en se tenant debout et avoir les mains vers le bas (au repos).

La station de mesure 100 pourrait toutefois avoir des formes et/ou des dimensions différentes, pourvu que la forme et/ou les dimensions permettent l'obtention des mesures présentement décrites. En particulier, la base 102 pourrait avoir une forme ovale ou plus arrondie dans le plan XY.

La station de mesure 100 peut avoir une masse comprise entre 3 et 6kg (par exemple 4 et 5kg).

Grâce au(x) capteur(s) de la base 102 et/ou au(x) capteur(s) de la poignée 110, la station de mesure 100 peut effectuer un groupe de mesures sur l'utilisateur. En particulier, les capteurs 104 utilisés comprennent des électrodes qui sont formées à partir de chemins conducteurs d'électricité montés sur le substrat 106 et/ou la poignée 110 (inserts métalliques, dépôts métalliques, etc.). Certaines mesures peuvent ne nécessiter que des capteurs de la base 102, d'autres mesures peuvent ne nécessiter que des capteurs de la poignée 110, d'autres mesures peuvent nécessiter simultanément des capteurs de la poignée 110 et de la base 102.

La station de mesure 100 peut effectuer un ECG à l'aide de la poignée 110 par exemple un ECG une voie), ou un ECG à l'aide de la poignée 110 et de la base 102 (par exemple un ECG multi-voie, comme un ECG six-voies). La station de mesure 100 peut aussi effectuer une analyse d'impédancemétrie corporelle BIA à l'aide de la poignée 110 et/ou de la base (BIA entre les jambes et/ou BIA segmentale). La station de mesure peut effectuer un IPG dans l'arc de jambes (« between legs ») ou IPG dans le pied (« in the foot »).

Les capteurs peuvent comprendre des électrodes aptes : à mesurer et/ou appliquer une tension (continue ou alternative) et/ou un potentiel (continu ou alternatif), et/ou injecter et/ou récupérer un courant (continu ou alternatif). Les fonctions de ces électrodes peuvent être choisies parmi la liste suivante : i+ et i-, pour l'injection de courant alternatif dans le corps d'un utilisateur ; V+ et V- pour mesurer une différence de potentiel dans le corps d'un utilisateur ; RA, LA et LL, pour mesurer un courant électrique. Les électrodes i+ et i-, V+ et V- sont utilisées pour un BIA, un IPG ou un ICG ; les électrodes RA, LL, LL sont utilisées pour un ECG.

En particulier, la station de mesure 100 est configuré pour réaliser différentes mesures. Le nombre d'électrodes étant limité (pour des considérations de surface et de nombre), la station de mesure 100 peut présenter un agencement particulier d'électrodes.

Comme indiqué précédemment, les capteurs peuvent comprendre des cellules de charge, grâce auxquelles la station de mesure 100 peut mesurer un poids et effectuer un BCG.

La poignée 110 est illustrée en détail en figure 4. La poignée 110 permet à la station de mesure 100 de réaliser une plus grande variété de mesures ou bien des mesures plus complètes, grâce à une connexion électrique avec au moins une main, voire les deux mains. En particulier, la BIA segmentale et/ou l'ECG multi-voies sont rendus possibles par l'ajout de la poignée 110 à la base 102. Les capteurs 402 de la poignée 110 comprennent par exemple des électrodes aptes à mesurer et/ou appliquer une tension et/ou un potentiel et/ou injecter et/ou récupérer un courant.

Dans un mode de réalisation, la poignée 110 comprend quatre électrodes, agencées en deux paires : une paire pour la main gauche et une paire pour la main droite. A cet effet, on nomme les électrodes de la poignée par : électrodes LH1, LH2, côte à côte sur une partie gauche de la poignée 110 et électrodes RH1, RH2, côte à côte sur une partie droite de la poignée (par partie gauche, respectivement droite, il est entendu la partie de la poignée destinée à être au contact de la main gauche, respectivement droite). « Côte à côte » signifie ici avec un espace entre les électrodes, pour isoler les électrodes les unes des autres. Les électrodes sont donc disposées à la suite les unes des autres entre deux extrémités de la poignée 110. Lorsque la poignée 110 est rectiligne, les électrodes sont disposées à la suite le long de la direction principale de la poignée 110. Les électrodes LH1 et RH1 sont positionnées axialement du côté d'une extrémité de la poignée ; les électrodes LH2, RH2 sont positionnées axialement du côté du centre de la poignée. On a donc, dans l'ordre, les électrodes suivantes : LH1, LH2, RH2, RH1.

Dans un mode de réalisation, les capteurs 402 de la poignée 110, lorsqu'ils sont des électrodes sont réalisés sous la forme de plusieurs inserts métalliques dans la poignée 110. Parmi les matériaux utilisables pour les inserts métalliques, on peut citer l'acier inoxydable, le titane, le laiton, l'ITO (oxyde d'indium-étain), nickel (ou alliage de nickel), ou des plastiques conducteurs. Pour le traitement et/ou l'acquisition de signaux, en particulier de l'ECG, la poignée 110 peut comprendre de l'électronique de traitement (amplification, filtrage, etc.), notamment pour l'ECG. Il est généralement préférable d'amplifier le signal au plus près des électrodes car le câble peut capter du bruit ambiant.

Pour réaliser une BIA segmentale modulable (pour obtenir des données sur les deux bras), quatre électrodes 402 sont nécessaires. Pour réaliser un ECG, deux électrodes 402 sont nécessaires.

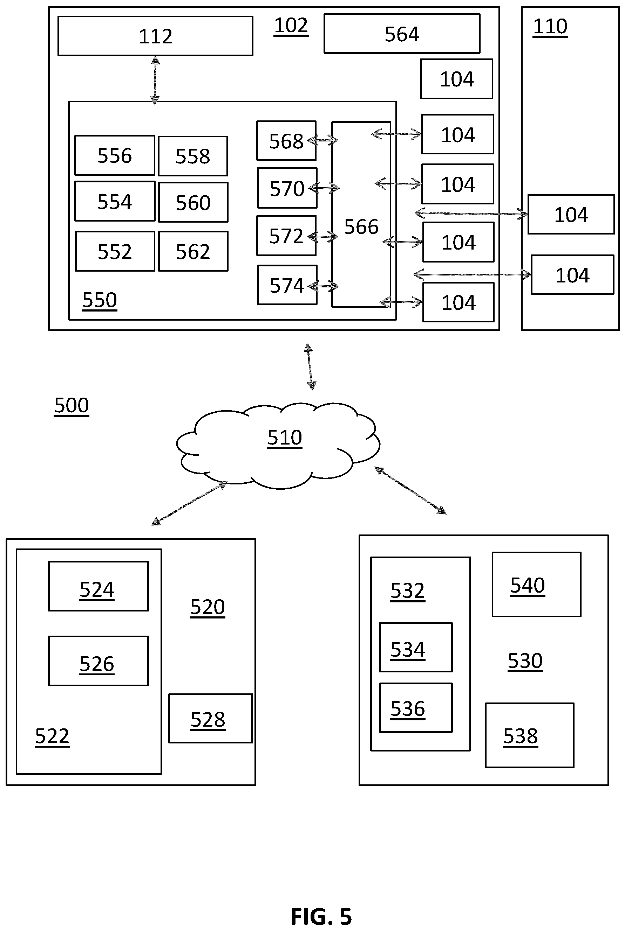

La figure 5 illustre une vue schématique de l'architecture globale 500 dans laquelle la station de mesure 100 peut être insérée. Cette architecture globale forme un système comprenant la station de mesure 100. En particulier, la station de mesure 100 peut communiquer avec des dispositifs tiers via un réseau de communication 510, qui est par exemple un réseau sans-fil (notamment un réseau compatible avec au moins l'un des protocoles de communication suivants : BlueTooth, Wi-Fi, cellulaire, etc.). Les dispositifs tiers peuvent comprendre un serveur 520 et un terminal mobile 530 (smartphone, etc.). Le serveur 520 peut comprendre une circuiterie de contrôle 522, incluant un processeur 524 et une mémoire 526, et une interface entrée/sortie (« input/output », I/O) 228, qui permet à la circuiterie de contrôle de recevoir et d'envoyer des données vers le réseau de communication 510. Le terminal mobile 530 peut comprendre une circuiterie de contrôle 532, incluant un processeur 234 et une mémoire 236, un comprendre une interface entrée/sortie (« input/output », I/O)538, qui permet à la circuiterie de contrôle de recevoir et d'envoyer des données. Le serveur 520 est un serveur distant, par exemple situé dans un centre de données (data center). Le terminal mobile 530 comprend en outre une interface d'utilisateur 540 (« user interface », UI) configurée pour afficher des informations à l'utilisateur et lui permettre, le cas échant, d'entrer des informations (comme la taille, le sexe, etc.). En particulier, la circuiterie de contrôle 532 est configurée pour faire fonctionner une application gérant l'environnement de la station de mesure 100. Le terminal mobile 530 est un objet personnel de l'utilisateur, généralement à proximité de ce dernier.

La station de mesure 100 peut communiquer avec le serveur 520 et/ou le terminal mobile 530. Dans un mode de réalisation, la station de mesure 100 peut communiquer directement avec le terminal mobile 530, par exemple via Bluetooth ou Bluetooth Low Emission (BLE). Cette communication peut être mise à en oeuvre à l'installation de l'appareil de mesure 100, notamment pour l'appairer avec le terminal mobile 530 et/ou pour configure une connexion au serveur 520 qui ne transite pas par le terminal mobile 530 et/ou en en secours d'une communication avec le serveur 520 défaillante. Dans un mode de réalisation, la station de mesure 100 peut communiquer directement avec le serveur 520, sans transiter par le terminal mobile 530. Cette communication permet à l'utilisateur d'utiliser la station de mesure même sans avoir son terminal mobile 530 à proximité.

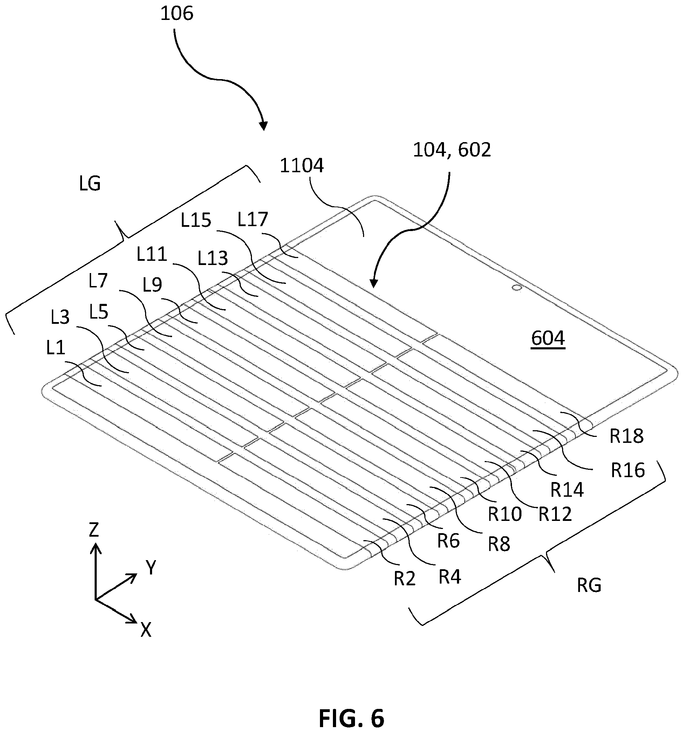

La station de mesure 100 comprend elle aussi une circuiterie de contrôle 550 avec un processeur 552 et une mémoire 554, et une interface entrée/sortie 556 (« input/output », I/O), qui permet notamment à la circuiterie de contrôle de recevoir et d'envoyer des données au réseau de communication 510. Le processeur 552 est configuré pour notamment traiter des données obtenues par les capteurs 104. En particulier, le processeur 552 peut exécuter des instructions d'un programme stocké dans la mémoire 554. La circuiterie de contrôle 550 peut comprendre un microcontrôleur, qui intègre le processeur 552, la mémoire 554 et l'interface entrée/sortie 556. La circuiterie de contrôle 550 peut en outre comprendre un dispositif frontal analogique (« *Analog Front End* », AFE). La circuiterie de contrôle 550 peut aussi comprendre un convertisseur analogique/digital (« *Analog to Digital Converters* », ADC). La station de mesure 100 comprend une source de tension (par exemple continue) 558 et une source de courant 560 (par exemple alternatif). La station de mesure 100 comprend aussi un voltmètre 562 (ou tout système permet de mesurer une tension). Le voltmètre 562 peut être intégré à l'AFE. La source de courant 560 peut être intégré à l'AFE et la source de tension 558 peut être intégrée au microcontrôleur MCU (par exemple via un convertisseur digital/analogique, « *digital to analog converter* » DAC). Certains capteurs 104 (en particulier les capteurs 402 de la poignée de la figure 4 ou les électrodes 602 de la base 102 sur la figure 6) sont reliés à la circuiterie de contrôle 550 (par exemple au MCU ou à l'AFE). La station de mesure 100 comprend une batterie 564, apte à alimenter en énergie les différents composants de la station de mesure 100.

La circuiterie de contrôle 550 et d'autres composants électroniques peuvent être montés sur un circuit imprimé PCB (*« printed circuit board »*)*,* par exemple attaché à la plaque de support 202. Des connecteurs relient les chemins conducteurs d'électricité de la plaque de mesure au PCB. Afin de pouvoir modifier les connexions des électrodes aux différents composants de la station de mesure 100, la station de mesure comprend un commutateur 566. Le commutateur 566, qui peut comprendre une pluralité d'interrupteurs pilotés par le MCU, sera décrit plus en détail par la suite).

La circuiterie de contrôle 550 comprend par exemple un système d'acquisition d'ECG, un système d'impédancemétrie (pour BIA ou IPG), et notamment un système d'ESC (pour évaluer la fonction sudorale de la peau, en effectuant un ESC « *electrochemical skin conductance* » ). De plus, la circuiterie de contrôle 550 peut comprendre un système de détection de présence de l'utilisateur, pour évaluer si le corps d'un utilisateur est bien en contact avec les électrodes. Le système d'acquisition d'ECG comprend des électrodes (représentées par 602 sur la figure 6 et 402 sur la figure 4) et un circuit électrique ECG 568 (qui intègre notamment différents étages d'amplification et/ou de filtrage et un démodulateur) ; le système d'impédancemétrie comprend notamment des électrodes (représentées par 602 sur la figure 6 et 402 sur la figure 4), la source de courant 560, le voltmètre 562 et un circuit électrique d'impédancemétrie 570 qui relie les électrodes à la source de courant et au voltmètre (qui intègre différents étages d'amplification et/ou de filtrage) ; le système d'ESC comprend des électrodes, la source de tension 558, et un circuit électrique d'ESC 572 (qui intègre différents composants électroniques, dont des résistances) ; le système de détection comprend des électrodes, la source de tension 558, le voltmètre 562 et un circuit électrique de détection 574 (qui relie les électrodes à la source de courant et au voltmètre (qui intègre différents étages d'amplification et/ou de filtrage) . Le commutateur 566 permet de relier les électrodes notamment aux différents circuits 568, 570, 572, 574 précités, ou bien de déconnecter toutes les électrodes de la circuiterie de contrôle 550.

La circuiterie de contrôle 550 se trouve essentiellement dans la base 102, à l'exception de notamment quelques composants (amplification, filtrage et interrupteurs) disposés dans un PCB dans la poignée 110, pour traiter les signaux avant de les faire transiter par le câble 302.

Comme indiqué auparavant, la station de mesure 100 comprend aussi l'affichage 114, telle qu'un écran (OLED/PMOLD, Retina, etc.), pour afficher à l'utilisateur des informations. Alternativement, la station de mesure 100 ne comprend pas d'affichage.

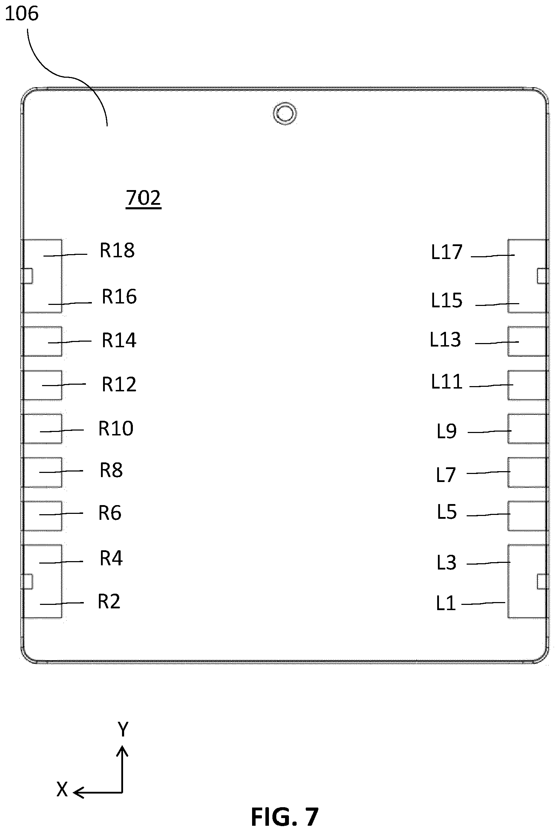

Dans un mode de réalisation, les capteurs 104 incluent des chemins conducteurs d'électricité 602 (appelés « électrodes ») sur la base 102 (voir notamment figures 6 et 7). Les électrodes 602 peuvent prendre la forme d'un dépôt métallique sur au moins une face supérieure 604 de la plaque de mesure 106. La face supérieure 604 de la plaque de meure 106 est définie comme la face recevant les pieds de l'utilisateur (la face externe qui est visible). Pour assurer la connexion électrique avec le PCB, les électrodes 602 peuvent passer par un bord de la plaque de mesure 106 et s'étendre jusque sur une face inférieure 702 de la plaque de mesure 106. Le bord (ou les bords concernés) de la plaque de mesure 106 peut présenter une forme arrondie pour assurer que le dépôt métallique se fasse bien et que la continuité électrique soit assurée. De plus, un bord arrondi permet d'éviter les risques de blessure lors de la préhension de la station de mesure 100. Par arrondi, il est signifié un arc de cercle ou des formes similaires. Le bord arrondi permet aussi de simplifier le dépôt métallique lors de la fabrication. La demande FR2106653, décrit en détail ces chemins conducteurs d'électricité.

Les électrodes 602 sont connectées au PCB via un connecteur électrique, qui permet de faire la connexion entre le chemin conducteur d'électricité sur la face inférieure 702 et le PCB monté sur la plaque de support 202. Le commutateur 566 permet de connecter et déconnecter les électrodes aux différents systèmes (système d'acquisition ECG, système d'impédancemétrie, système de sudogramme, etc.). De la sorte, chaque électrode peut avoir plusieurs fonctions différentes en fonction de la position de commutation du commutateur 566. Le commutateur 566 comprend par exemple une pluralité d'interrupteurs pilotés par le MCU.

La face supérieure 604 de la base 102 comprend un groupe gauche LG d'électrodes (destiné à être en contact avec le pied gauche, et un groupe droit RG d'électrodes destiné à être en contact avec le pied droit. Lorsque la base 102 est posée en condition normale d'utilisation, l'utilisateur met ses pieds sur une partie gauche de la balance et une partie droite de la balance (les doigts de pied étant du côté de l'affichage 114). Les figures 6 et 7 représentent des chemins conducteurs d'électricité L1, L3, L5, L7, L9, L11, L13, L15, L17 qui forment les électrodes du groupe gauche LG d'électrodes et des chemins conducteurs d'électricité R2, R4, R6, R8, R10, R12, R14, R16, R18, qui forment les électrodes du groupe droite RG d'électrodes.

Les électrodes 602 peuvent prendre la forme de bandes parallèles les unes aux autres le long de la direction X (les bandes s'étendent le long de la largeur X de la base 102).

Dans l'architecture illustrée, les couples de chemins conducteurs d'électricité L1 et L3 ; L15 et L17 ; R2 et R4 ; R16 et R18 ne sont pas indépendants mais sont reliés électriquement en permanence, de sorte que la base 102 comprend en pratique sept électrodes indépendantes dans le groupe gauche LG et sept électrodes indépendantes dans le groupe droit RG. Ces connexions électriques permanentes peuvent se faire via les chemins électriques sur la plaque de mesure 106 (par exemple sur la face inférieure 702, non-illustré) ou via le PCB de la station de mesure 100.

Dans un exemple, les chemins conducteurs d'électricité de la face supérieure 604 correspondant aux électrodes ont une dimension (sur la face supérieure 604) selon la longueur Y comprise entre 1,5cm et 2 cm (par exemple 1,7cm) ; l'espacement entre deux bandes peut être compris entre 0,5cm et 1cm (par exemple 0,85cm) ; les électrodes peuvent avoir une dimension selon la largeur X supérieure à 10cm.

En particulier, chaque groupe LG, RG peut comprendre au moins quatre électrodes indépendantes pour notamment pouvoir effectuer un IPG dans le pied (deux électrodes connectées à la source de courant alternatif 560 et deux électrodes connectées au voltmètre 562). Dans un autre mode de réalisation, chaque groupe LR, RG peut comprendre au moins deux électrodes indépendantes (pour effectuer un ESC avec anode/cathode et une électrode haute impédance, ou pour effectuer un BIA ou un IPG entre les jambes), ou trois électrodes indépendantes.

Dans un mode de réalisation, la station de mesure 100 peut effectuer un électrocardiogramme, ECG. Pour cela, les capteurs 104 de la station de mesure 100 (base 102 et poignée 110) peuvent comprendre une pluralité de surfaces conductrices permettant de détecter des courants électriques traversant le corps d'un utilisateur. La station de mesure 100 comprend un système d'acquisition d'électrocardiogramme ECG, mettant en oeuvre certains des capteurs 104.

Sur la base 102 illustrée notamment en figure 6, ces surfaces conductrices prennent la forme des bande parallèles L1 à L17 et R2 à R18. En particulier, la base 102 comprend deux groupes d'électrodes : un premier groupe d'électrode LG, qui est positionné sur une partie gauche de la base 102 en utilisation normale (appelé groupe gauche, mais ce terme ne doit pas être interprété limitativement pour un pied gauche uniquement), et un deuxième groupe d'électrodes RD, qui est positionné sur une partie droite de la base 102 en utilisation normale (appelé groupe droite, mais ce terme ne doit pas être interprété limitativement pour un pied droit uniquement). En particulier, un utilisateur pourrait monter à l'envers sur la base 102 (pied gauche sur le groupe droit RG et pied droit sur le groupe gauche LG). Les figures 1 et 6 illustrent un exemple d'un mode de réalisation des groupes droite et gauche LG, RG.

Sur la poignée, ces surfaces conductrices prennent la forme de quatre électrodes RH1, RH2, LH2, LH1. En configuration ECG, les électrodes RH1, RH2 et LH1, LH2 peuvent être connectées deux à deux, de sorte que la poignée comprennent deux électrodes RA (« right arm »), LA (« left arm »). Alternativement, une seule électrode parmi les électrodes RH1, RH2 et une seule électrode parmi les électrodes LH1, LH2 peut être connecté au système d'acquisition ECG.

Grâce à ces capteurs 104, la station de mesure ECG peut effectuer un ECG multi-voie en utilisant trois électrodes de mesure : une électrode main gauche (électrode LH), une électrode main droite (électrode RH) et une électrode dans le pied, par exemple dans le pied gauche (électrode LL). L'électrode LL peut être formée par au moins une bande du groupe LG (par exemple les bandes L15, L17 de la base 102).

Dans un mode de réalisation, le système d'acquisition ECG 1010 peut mesurer les voies DII (entre les électrodes RA et LL) et DIII (entre les électrodes LA et LL) et peut calculer les autres voies (DI, aVF, aVL et aVR).

Alternativement, la station de mesure 100 peut effectuer une ECG mono-voie en utilisant deux électrodes de mesure : par exemple entre les bras avec un électrode main gauche (électrode LH) et une électrode main droite (électrode RH) ; par exemple entre les jambes avec une électrode pied gauche (électrode LL) et une électrode pied droite (électrode RL). L'électrode RL peut être formée par au moins une bande du groupe RG (par exemple les bandes R16, R18 de la base 102).

Lors d'un ECG, le corps de l'utilisateur peut être mis à une masse virtuelle afin d'éviter que les amplificateurs opérationnels du système d'acquisition ECG ne saturent. Par exemple, l'électrode LL peut servir à fixer ce potentiel.

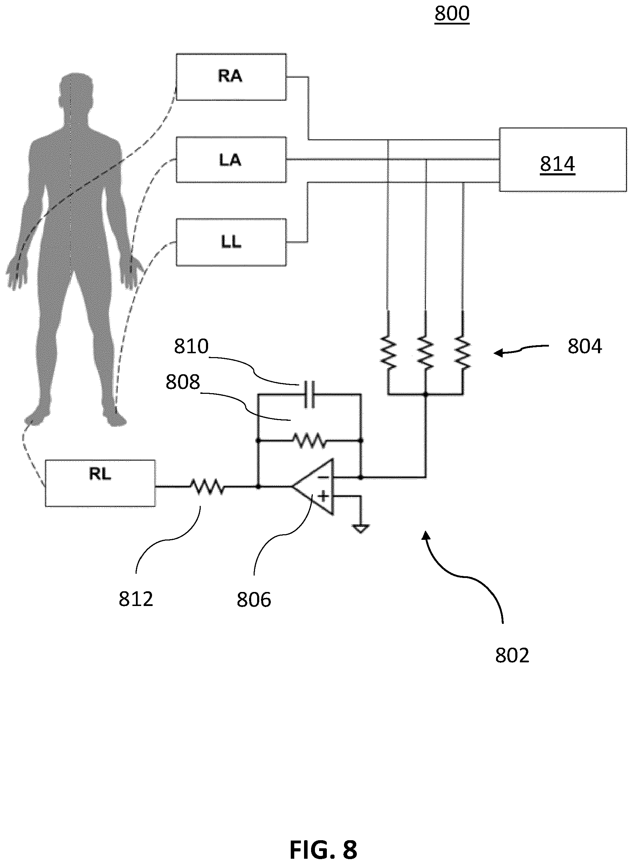

Dans un mode de réalisation, la station de mesure 100 comprend, dans un agencement d'ECG 800, un circuit RLD 802 (« *right-leg drive* »), illustré en figure 8. Le circuit RLD 802 est illustré dans le cas d'un ECG multi-voie avec les électrodes LA, RA et LL. Le principe consiste à mesurer la tension dite « *common-mode voltage Vcm* », puis de réinjecter une forme amplifiée et inversée dans le corps de cette tension mesurée. Cette tension Vcm est aussi appelée « *Wilson Common Terminal, WCT* ». Les trois potentiels des électrodes RA, LA, LL sont moyennés à l'aide de résistance(s) 804 puis envoyés vers l'entrée négative d'un amplificateur opérationnel 806. La masse virtuelle est envoyée vers l'entrée positive de l'amplificateur opérationnel 806 pour former une référence de potentiel à laquelle on souhaite mettre l'utilisateur. Une ligne de rétroaction avec une résistance de retour 808 (environ 1 MOhm) et un condensateur de retour 810 (pour fixer le gain en boucle ouverte, environ 1,5nF) est prévue entre l'entrée négative et la sortie de l'amplificateur opérationnel 806. Enfin, l'électrode RL est reliée à la sortie de l'amplificateur opérationnel 806 par une résistance 812 pour limiter le courant qui pourrait parcourir le corps de l'utilisateur. Les électrodes RA, LA, LL sont connectées à un circuit électronique ECG 814.

Le circuit RLD 802 décrit ci-dessus permet d'obtenir un signal ECG de meilleure qualité par rapport aux dispositifs sans circuit RLD. Néanmoins, pour obtenir des mesures synchronisées, il peut être souhaitable d'effectuer un ECG en même temps qu'une autre mesure. Cette autre mesure peut impliquer l'injection d'un courant dans le corps de l'utilisateur : dans le cas, par exemple, d'un IPG.

Dans le cas d'un IPG, une source de courant injecte un courant dans le corps de l'utilisateur et un voltmètre mesure une chute de tension au niveau du corps induite par ce courant. Un système d'impédancemétrie comprend ainsi notamment la source de courant et une boucle de rétroaction pour maintenir la tension Vcm dans la plage de fonctionnement des amplificateurs.

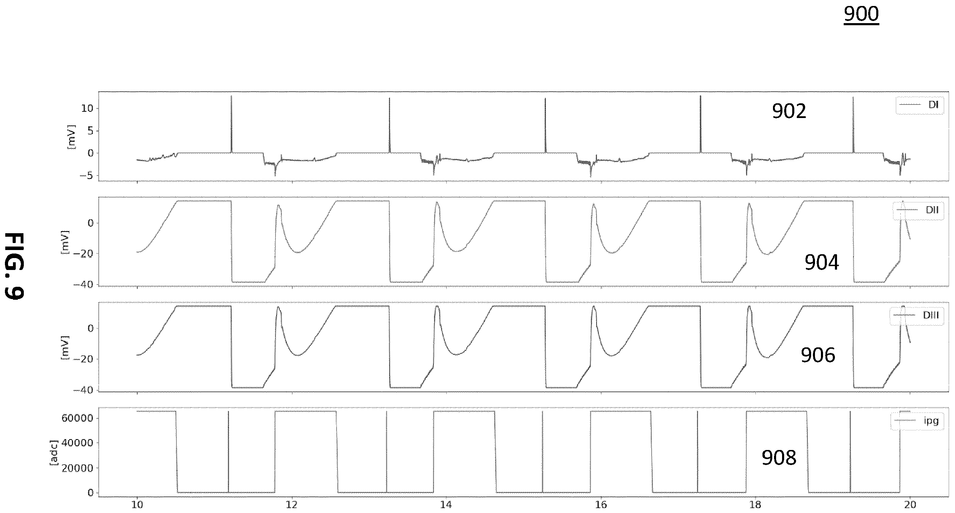

Lorsque l'IPG et l'ECG sont effectués en même temps, il y a ainsi deux boucles de rétroaction (celle du circuit RLD 802 et celle du système d'impédancemétrie) qui sont activées et qui génèrent des perturbations affectant les mesures effectuées (voire faisant saturer les amplificateurs opérationnels). La figure 9 illustre des résultats 900 d'un ECG avec RLD mesuré en même temps qu'une injection de courant pour IPG. Les voies DI (entre RA et LA) 902, DII (entre RA et LL) 904, DIII (entre LA et LL) 906 et la tension obtenue pour l'IPG 908 sont représentées. Les signaux ne sont pas exploitables : pour l'IPG, aucun pic de passage du sang n'est visible (saturation) et les signaux d'ECG sont également saturés.

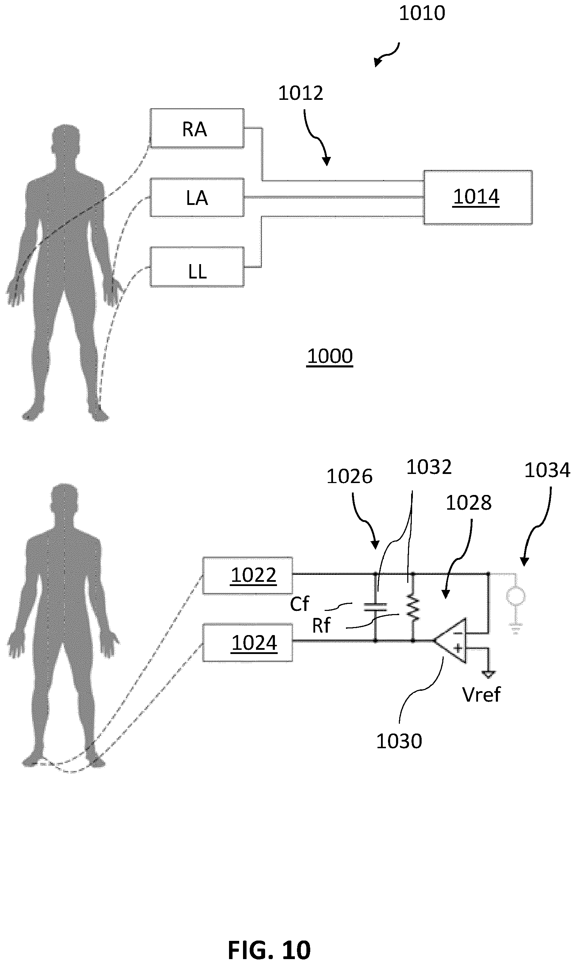

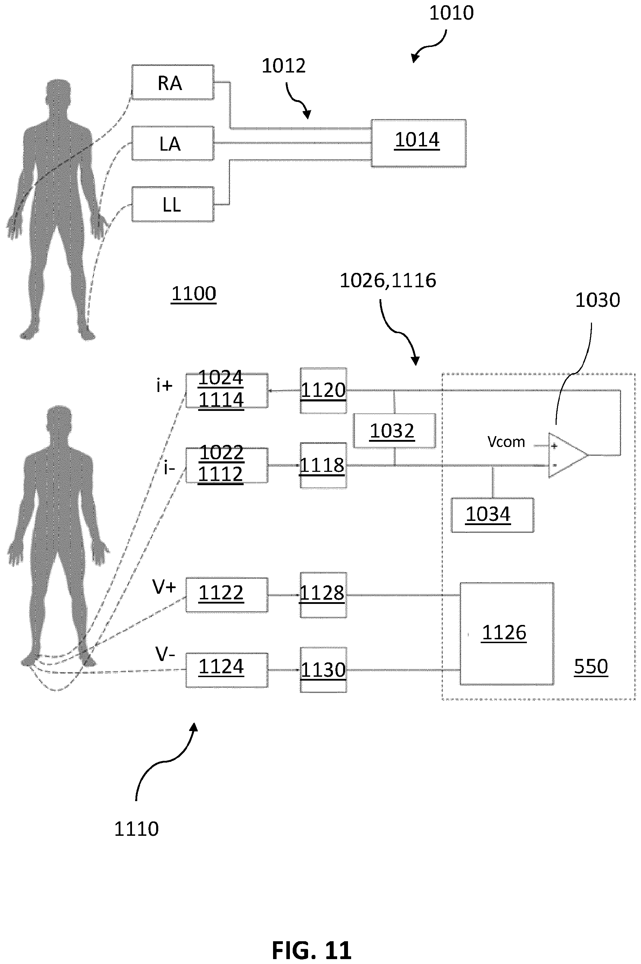

Dans un mode de réalisation, la station de mesure 100 comprend une alternative au circuit RLD qui rend l'acquisition d'un ECG compatible avec une mesure simultanée qui nécessite l'injection de courant dans le corps humain (par exemple une impédancemétrie). La figure 10 illustre une configuration 1000 d'un agencement alternatif au circuit RLD (il s'agit du même individu illustré en double pour plus de clarté) et la figure 11 illustre l'intégration de cette configuration 1000 dans un schéma 1100 qui illustre un système d'acquisition ECG 1010 et un système d'impédancemétrie 1110.

La station de mesure 100 comprend un système d'acquisition d'ECG 1010. Comme indiqué auparavant, ce système comprend une pluralité d'électrodes RA, LL, LL configurées pour être au contact de différentes parties du corps d'un utilisateur. En particulier, dans la configuration illustrée, la pluralité d'électrodes comprend l'électrode RA montée sur la poignée 110, l'électrode LA montée sur la poignée 110 et l'électrode LL, montée sur la base 102. Les électrodes RA, LA, LL sont connectées à un circuit électronique ECG 1012, qui comprend notamment une unité d'acquisition 1014 (AFE ou unité de contrôle).

La station de mesure 100 comprend en outre deux électrodes de contrôle 1022, 1024 configurées pour être au contact de l'utilisateur. Ces électrodes de contrôle 1022, 1024 peuvent être sur la base 102 de la station de mesure 100. Par exemple, les électrodes de contrôle 1022, 1024 sont formées par des chemins conducteurs d'électricité 602 de la base 102. Les deux électrodes de contrôle 1022, 1024 peuvent faire partie du groupe droit d'électrodes RG de la base 102 : l'électrode de contrôle 1022 peut comprendre au moins la bandes R16 (bande R16 et R18 par exemple) et l'électrode de contrôle 1024 peut comprendre au moins la bande R4 (bandes R2 et R4 par exemple). Dans le système d'acquisition d'ECG 1010, l'électrode LL est prise parmi les électrodes du groupe gauche LG de la base 102.

Les électrodes de contrôle 1022, 1024 pourraient être positionnées n'importe où sur le corps. Néanmoins, le choix de la base 102 pour ces deux électrodes de contrôle 1022, 1024 présente plusieurs intérêts : l'utilisateur est déjà au contact des électrodes de contrôle 1022, 1024 lorsqu'il s'installe sur la base 102 pour effectuer un ECG (pour être en contact avec l'électrode LL), et les électrodes de contrôle 1022, 1024 peuvent aussi servir d'injection de courant pour effectuer, par exemple, un IPG. Cela sera expliqué en détail par la suite.

La station de mesure 100 comprend en outre un circuit de connexion électrique 1026 qui comprend une boucle de rétroaction 1028 qui relie les deux électrodes de contrôle 1022, 1024.

L'une des deux électrodes de contrôle 1022, 1024 (l'électrode de contrôle 1022 dans la configuration 1000) permet d'obtenir le potentiel du corps de l'utilisateur. Ce potentiel est inversé et amplifié par la boucle de rétroaction 1028 puis réinjecté au corps par l'autre des deux électrodes de contrôle 1022, 1024 (l'électrode 1024 dans la configuration 1000). On obtient ainsi une stabilisation du potentiel du corps qui permet de stabiliser électriquement le système d'acquisition ECG.

Dans un mode de réalisation, la boucle de rétroaction 1028 comprend notamment un amplificateur opérationnel 1030 et une connexion de rétroaction 1032. La borne positive de l'amplificateur opérationnel 1030 est connectée à une tension de référence Vref et la borne négative de l'amplificateur opérationnel 1030 est connectée à l'électrode de contrôle 1022. La sortie de l'amplificateur opérationnel 1030 est connectée à l'électrode de contrôle 1024. La connexion de rétroaction 1032 relie la sortie de l'amplificateur opérationnel 1030 à l'entrée négative de l'amplificateur opérationnel 1030.

La connexion de rétroaction 1028 comprend au moins un composant passif (notamment une résistance, un condensateur, et/ou une bobine). Par exemple, l'au moins un composant passif peut être une résistance Rf qui forme le gain de la boucle de rétroaction 1028. La résistance Rf est choisie avec une valeur de résistance élevée (par exemple environ 10 MOhms). Une résistance Rf élevée peut néanmoins poser des difficultés de stabilisation du schéma électrique. Pour stabiliser, la connexion de rétroaction 1032 peut comprendre en plus un condensateur Cf, en parallèle de la résistance Rf. La valeur du condensateur Cf peut être d'environ 47 pF.

Dans cette configuration particulière du circuit de connexion électrique, l'électrode de contrôle 1022 permet d'obtenir le potentiel du corps de l'utilisateur et l'électrode de contrôle 1024 met le corps de l'utilisateur à un potentiel rétrocontrôlé.

Les électrodes de contrôle 1022, 1024 ne font pas partie de la pluralité d'électrodes ECG (i.e. en sont distinctes) : ce sont des électrodes indépendantes (c'est-à-dire électriquement indépendantes). La configuration 1000 met donc en oeuvre, pour une ECG multi-voie, cinq électrodes indépendantes : trois électrodes RA, LL, LL pour l'acquisition de l'ECG et deux électrodes de contrôle 1022, 1024 pour contrôler le potentiel du corps.

En outre, le circuit de connexion électrique 1026 est électriquement indépendant du circuit électronique ECG 1012. Par électriquement indépendant, il est signifié qu'au sein de la station de mesure 100 il n'y a pas de connexion électrique entre le circuit de connexion électrique 1026 et le circuit électronique ECG 1012. Des connexions communes à la masse de la station de mesure 100 ne sont pas considérées comme des connexions électriques. De la même façon, une connexion via le corps de l'utilisateur n'est pas considérée comme une connexion électrique.

Comme mentionné précédemment, un avantage de la configuration 1000 de la figure 10 est de pouvoir permettre, soit en simultané avec l'ECG, soit indépendamment, une mesure biophysique impliquant l'injection de courant dans le corps humain.

Dans un mode de réalisation, la station de mesure 100 comprend un système d'impédancemétrie 1110. Le système d'impédancemétrie comprend une source de courant 1034 et deux électrodes d'injection de courant 1112 (aussi notée i-) et 1114 (aussi notée i+) reliées à la source de courant 1034 par un circuit d'injection de courant 1116.

En particulier, le circuit d'injection de courant 1116 est le circuit de connexion électrique 1026 et les électrodes d'injection de courant 1112, 1114 sont les électrodes de contrôle 1022, 1024.

En effet, la linéarité du système autorise la superposition d'un circuit de stabilisation pour l'ECG et d'un système d'impédancemétrie 1110. Le circuit de connexion 1026 fait office de boucle de rétroaction pour l'injection de courant afin de contrôler l'amplitude du courant.

La source de courant 1034 peut comprendre un générateur de tension connecté à la masse d'une part et à la borne négative de l'amplificateur opérationnel 1030 via un circuit RC (résistance et condensateur en série) d'autre part. La source de courant 1034 peut être intégrée à l'AFE.

Dans une configuration, la source de courant 1034 est une source de courant alternatif (par exemple une source de tension alternative en série avec un condensateur et une résistance). Une tension de mode-commun (« *common-mode voltage* ») peut alors exister. La boucle de rétroaction 1028, et en particulier la connexion de rétroaction 1032, permet de s'assurer que l'amplificateur opérationnel 1030 fonctionne dans sa plage de valeurs en autorisant une boucle pour le courant continu.

Pour éviter l'injection de composantes basse-fréquence ou l'injection d'un courant continu généré par l'amplificateur opérationnel 1034, des filtres passe-haut 1118, 1120 sont positionnés entre les électrodes d'injection de courant 1112, 1114 et le circuit d'injection de courant 1116. Les filtres passe-haut 1118, 1120 peuvent comprendre des condensateurs et/ou des résistances. Dans un mode de réalisation, le filtre passe-haut 1118 comprend un condensateur et le filtre passe-haut 1120 comprend en parallèle un condensateur et une résistance. Dans un autre mode de réalisation, les deux filtres passe-haut 1118, 1120 comprennent chacun en parallèle un condensateur et une résistance.

La fréquence du courant alternatif injecté peut être comprise entre 5kHz et 1000 kHz.

Afin de pouvoir solliciter le système d'acquisition d'ECG 1010 sans solliciter le système d'impédancemétrie 1110, la source de courant 1034 peut être pilotable (pour être activée ou désactivée), par exemple via l'AFE directement, ou bien un interrupteur peut être positionné entre la source de courant 1034 et le circuit d'injection de courant 1116, à l'aide par exemple du commutateur 566 et/ou de la circuiterie de contrôle 550.

Le système d'impédancemétrie 1100 comprend aussi deux électrodes de mesure 1122, 1124 (aussi notées V+, V-, respectivement) configurées pour mesurer un potentiel du corps humain, et un voltmètre 1126, relié aux deux électrodes de mesure 1122, 1124.

Pour s'assurer que le voltmètre 1126 ne mesure que des composantes haute-fréquence, des filtres passe-haut 1128, 1130 sont positionnés entre les électrodes de mesure 1122, 1124 et le voltmètre 1126. Les filtres passe-haut 1128, 1130 peuvent comprendre des condensateurs.

Les électrodes de mesure 1122, 1124 peuvent être montées sur la base 102, et en particulier dans le même groupe d'électrodes RG que les électrodes d'injection de courant 1112, 1114. Dans une configuration privilégiée, les électrodes de mesure 1122, 1124 sont situées entre les électrodes d'injection de courant 1112, 1114. Par exemple, les chemins conducteurs R14 et R6 peuvent être utilisés respectivement pour les électrodes de mesure 1122, 1124. Le système d'impédancemétrie 1110 permet de faire un IPG dit dans le pied : la variation d'impédance du pied peut ainsi être mesurée.

Alternativement, en choisissant une électrode d'injection 1112, 1114 et une électrode de mesure 1122, 1124 dans le groupe gauche d'électrodes LG et une électrode d'injection 1112, 1114 et une électrode de mesure 1122, 1124 dans le groupe droit d'électrodes RG, le système d'impédancemétrie 1100 permet de faire un IPG dit dans l'arc de jambe (« between legs ») : la variation d'impédance de l'entrejambe peut ainsi être mesurée. Par exemple, le chemin conducteur L13 peut former l'électrode d'injection de courant 1112 (qui est aussi l'électrode de contrôle 1022), un ou plusieurs chemins conducteurs parmi L1, L3, L5, L7 peut former l'électrode de mesure 1124, un ou plusieurs chemins conducteurs parmi R14, R16, R18 peut former l'électrode d'injection de courant 1114 (qui est aussi l'électrode de contrôle 1024), un ou plusieurs chemins conducteurs parmi R2, R4, R6, R8 peut former l'électrode de mesure 1122. Un ou plusieurs chemins conducteurs parmi les chemins conducteurs L15, L17 peut former l'électrode LL. Les électrodes RA et LA sont sur la poignée 110.

Plus généralement, à la place du système d'impédancemétrie, la station de mesure 100 peut comprendre un système de mesure impliquant l'injection d'un courant et/ou l'application d'une tension. La source de courant peut être alternative (IPG, BIA) et/ou la source de tension peut être continue (mesure de l'activité sudorale, décrite dans les documents WO2006/136598, WO2008/107324, WO2013/075963, WO2014/033105, WO2015/036530, WO2016/083432).

Dans une variante, une électrode du système d'acquisition ECG est commune avec l'une des électrodes de contrôle.

L'acquisition d'un ECG et d'un IPG synchronisés permet d'obtenir notamment des données physiologiques relatives au fonctionnement cardiovasculaire d'un individu (activation du coeur, passage du sang dans les membres, etc.). La circuiterie de contrôle 550 peut piloter le système d'acquisition ECG 1010 et le système d'impédancemétrie 1110 pour pouvoir : soit lancer un ECG seul, soit lancer une analyse d'impédancemétrie seule (IPG, BIA, ...), soit un ECG et une analyse d'impédancemétrie en même temps.

Comme indiqué plus haut, la station de mesure 100 comprend en outre des cellules de charge apte à mesurer un poids de l'utilisateur. Les cellules de charge permettent aussi d'effectuer un BCG (ballistocardiogramme). De la même façon, la circuiterie de contrôle 550 peut effectuer de façon synchronisée un BCG, un ECG et un IPG. L'ECG peut alors servir à identifier des pics dans les signaux de BCG et l'IPG.

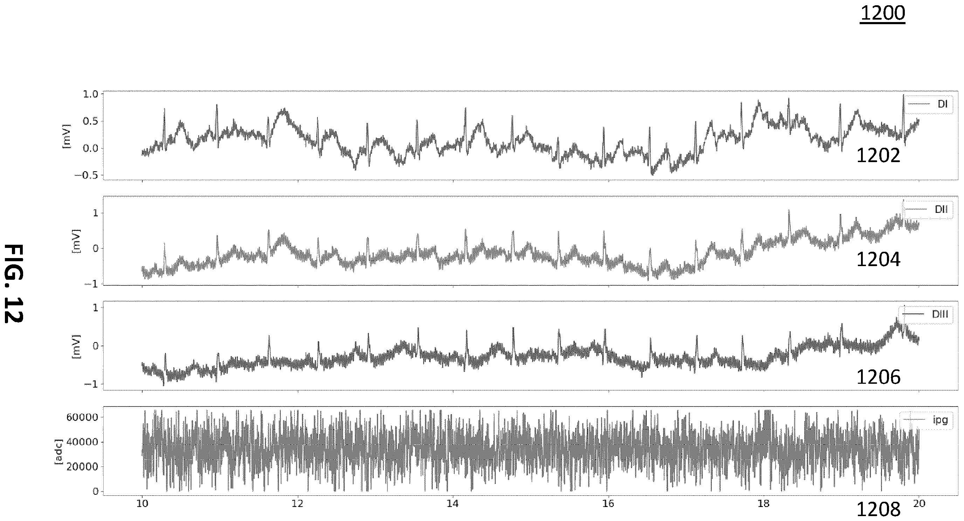

La figure 12 illustre des résultats 1200 d'un ECG avec la configuration de la figure 10 (sans acquisition simultanée d'un IPG, donc). Les voies DI (entre RA et LA) 1202, DII (entre RA et LL) 1204, DIII (entre LA et LL) 1206, et l'IPG 1208 (en pas d'ADC) sont représentées en synchronisés. Ces signaux sont exploitables, à l'exception évidente du signal récupéré pour l'IPG, puisque la source de courant 1034 n'était pas activée. Cette figure 12 montre que la boucle de rétroaction définie par les deux électrodes de contrôle 1022, 1024 permet de stabiliser l'ECG afin d'obtenir des signaux de bonne qualité.

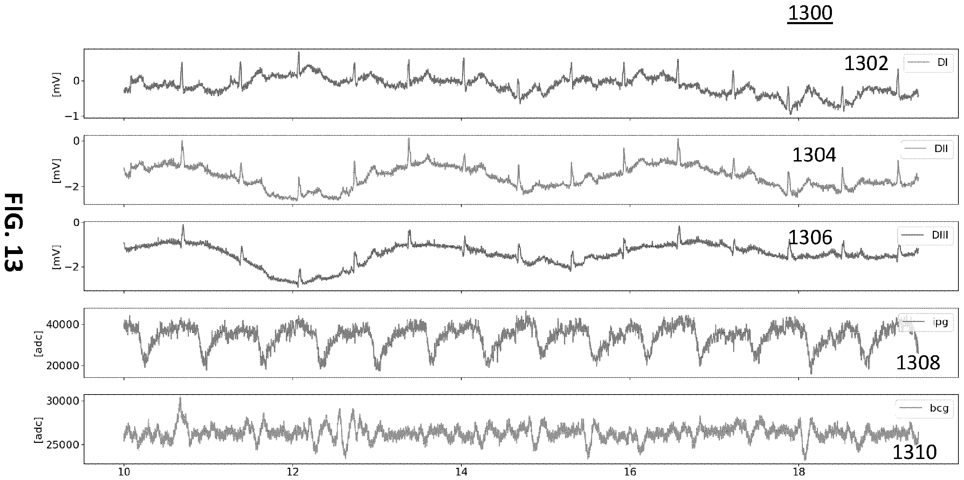

La figure 13 illustre des résultats 1300 d'un ECG effectué avec IPG en simultané, avec la configuration de la figure 11. Les voies DI (entre RA et LA) 1302, DII (entre RA et LL) 1304, DIII (entre LA et LL) 1306, l'IPG 1308 (en pas d'ADC), et un BCG 1310 (en pas d'ADC) sont représentées (synchronisés). Les mesures effectuées montrent qu'il n'y a pas de phénomène de saturation et les pics sont clairement identifiables. Ces signaux sont donc exploitables. Cette figure 13 montre qu'un IPG et un ECG peuvent être effectués en simultané. La figure 13 illustre en outre le signal de BCG, acquis simultanément.

La station de mesure 100 peut effectuer différentes mesures, comme du BIA (entre les jambes ou segmental avec la poignée). A cette fin, chaque chemin conducteur 602 peut être une électrode assurant différentes fonctions. Pour cela, la station de mesure 100 comprend un commutateur permettant de relier les chemins conducteurs 602 à différents composants.

Dans un exemple (ECG et IPG en arc-de-jambe), les bandes L15 et L17 forment l'électrode (qui doit donc être reliée au circuit électronique ECG 1012), la bande L13 forme l'électrode d'injection de courant 1112 (qui doit donc être reliée au circuit de connexion électrique 1026), les bandes L14, L16, L18 forment l'électrode d'injection de courant 1114 (qui doit donc être reliée au circuit de connexion électrique 1026), les bandes L1, L3, L5, L7 forment l'électrode de mesure 1124 (qui doit donc être reliée au voltmètre 1126), les bandes R2, R4, R6, R8 forment l'électrode de mesure 1122 (qui doit donc être reliée au voltmètre 1126).

Dans un autre exemple (ECG et IPG dans le pied), les bande L15 et L17 forment l'électrode LL (qui doit donc être reliée au circuit électronique ECG 1012), les bandes L16, L18 forment l'électrode d'injection de courant 1112 (qui doit donc être reliée au circuit de connexion électrique 1026), les bandes L2, L4 forment l'électrode d'injection de courant 1114 (qui doit donc être reliée au circuit de connexion électrique 1026), la bande L14 forme l'électrode de mesure 1124 (qui doit donc être reliée au voltmètre 1126), la bande L6 forme l'électrode de mesure 1122 (qui doit donc être reliée au voltmètre 1126).

Le commutateur 566 permet notamment de commuter entre ces deux configurations. En particulier, il permet de connecter différentes électrodes au circuit de connexion électrique 1026, en fonction de la mesure désirée.

De plus, pour faire un BIA segmental ou pour faire un IPG pied gauche, d'autres configurations des groupes gauche LG et droit RG d'électrodes sont possibles, dans lesquelles certaines électrodes doivent être connectées ou déconnectées du circuit de connexion électrique 1026.

Dans un mode de réalisation, la station de mesure permet d'évaluer la fonction sudorale de la peau (mesure ESC) en excitant les électrodes 602 avec des paliers de tension continu et constante. Les demandes de brevet WO2006/136598, WO2008/107324, WO2013/075963, WO2014/033105, WO2015/036530 WO2016/083432 explique ce fonctionnement. Dans ce mode de réalisation, la plupart des électrodes 602 (toutes les bandes de chaque groupe d'électrodes LG, RG sauf une, qui peut être reliée à une haute impédance), sont connectées à la source tension continue.

Le commutateur 566 permet de commuter entre toutes ces différentes configurations.

La station de mesure 100 comprend en outre des cellules de charge apte à mesurer un poids de l'utilisateur. Les cellules de charge permettent aussi d'effectuer un BCG (ballistocardiogramme). De la même façon, la circuiterie de contrôle 550 peut effectuer de façon synchronisée un BCG, un ECG et un IPG. L'ECG peut alors servir à identifier les pics dans le BCG et l'IPG.

Dans un mode de réalisation, la station de mesure 100 comprend un système de détection de présence de l'utilisateur (ci-après « système de détection »). Ce système de détection permet notamment de détecter que l'utilisateur est en contact simultanément avec une électrode de la poignée 110 (ci-après appelé « électrode de main ») et avec une électrode de la base 102 (ci-après appelée « électrode de pied »). On pourra parler de « détermination de contact ».

Un tel système permet d'éviter de lancer des mesures nécessitant la poignée et qui seront nécessairement non concluant.

Le système de détection peut générer une donnée indiquant que le contact est effectif ou que le contact n'est pas effectif. Alternativement, le système de détection ne génère pas de données en l'absence de contact avec le pied et la main.

Le système de détection fait partie de la circuiterie de contrôle 550 de la station de mesure 100.

Le système de détection peut comprendre des électrodes (dont l'électrode de pied et l'électrode de main précitées), un générateur de tension (par exemple la source de tension 558), un voltmètre (par exemple le voltmètre 562) et un circuit de détection 574. Dans un mode de réalisation, l'électrode de pied est mise à un potentiel prédéterminé par le générateur de tension (par exemple entre 0,5V et 2V) : s'il y a un contact entre l'électrode de pied et le corps de l'utilisateur, ce dernier va être mis au potentiel prédéterminé. Le voltmètre permet de mesurer le potentiel de l'électrode de main : s'il y a un contact entre l'électrode de main et le corps de l'utilisateur, l'électrode de main mesure un potentiel proche du potentiel prédéterminé. Aux fins de la détection, un seuil prédéterminé est choisi ; le seuil prédéterminé correspond au potentiel prédéterminé moins un écart pouvant correspondre aux pertes de potentiel du corps. Par exemple, si le potentiel prédéterminé est de 2V, le seuil peut être de 1,5V.

En réponse à une mesure d'un potentiel de l'électrode de main supérieur au seuil, le système de détection indique que la détection a eu, et donc que l'utilisateur est bien en position sur la base 102 et qu'il tient la poignée 110. Les différentes mesures de la station de mesure 100 qui nécessite l'utilisation de la poignée peuvent alors être effectuées.

On définit un ou plusieurs systèmes d'acquisition de mesure par la poignée, qui correspondent à des systèmes d'acquisition de mesure qui nécessite la poignée : le système d'acquisition d'ECG en fait partie, le système d'impédancemétrie pour faire de la BIA segmentale en fait partie.

L'électrode de pied et l'électrode de main peuvent être des électrodes utilisées par un système d'acquisition de mesure par la poignée, tel que le système d'acquisition d'ECG et/ou par un système d'impédancemétrie.

Dans un mode de réalisation, le système d'acquisition de mesure par la poignée est activé en réponse à une détection de contact simultané par le système de détection. En d'autres termes, la circuiterie de contrôle 550, sur réception d'une détection de contact simultané par le système de mesure, déclenche les mesures nécessitant la poignée ou interrompt le déclenchement de mesure nécessitant la poignée.

La circuiterie de contrôle 550, notamment la mémoire 526, peut comprendre une séquence de mesures à effectuer par différent systèmes d'acquisition de mesure (poids, ECG, BIA, IPG, ESC, etc.). Certaines de ces mesures nécessitent la poignée. En réponse à une absence de détection de contact simultané, la circuiterie de contrôle peut modifier la séquence de mesures afin que les mesures nécessitant la poignée ne soient pas mises en oeuvre. Complémentairement ou alternativement, en réponse à une détection de contact simultané, la circuiterie de contrôle peut modifier la séquence de mesures afin que les mesures nécessitant la poignée soient mises en oeuvre.

Dans une séquence de mesures successives M1, M2, M3, M4, où M2 nécessite la poignée 110, la circuiterie de contrôle 550 peut générer la séquence M1, M3, M4 en réponse à l'une absence de détection de contact simultané.

Par exemple, le système d'acquisition d'ECG peut être mis en oeuvre en réponse à une détection de contact simultané par le système de détection.

L'utilisateur peut décider volontairement de ne pas saisir la poignée : grâce au système de détection, la séquence de mesures peut être significativement moins longue puisque les mesures utilisant la poignée peuvent être supprimées de la séquence

Dans un mode de réalisation, la base 102 comprend un système de mesure de poids (par exemple avec un capteur de poids tel que décrit auparavant). Le système de détection peut être activé en réponse d'une détection de poids par le système de mesure de poids. Cela permet donc d'activer le système de détection uniquement quand un utilisateur est présent sur la balance, afin de préserver la batterie de la station de mesure 100. En particulier, la séquence de mesure peut être déclenchée par la détection de poids par le système de mesure de poids (la première mesure pouvant être la mesure du poids elle-même).

Dans un mode de réalisation, la circuiterie de contrôle 550 (et en particulier la mémoire) stocke une séquence complète de mesure, qui comprend toutes les mesures permises par la station de mesure 100, dont les mesures nécessitant la poignée 110, et stocke une séquence partielle de mesure, qui comprend au plus toutes les mesures ne nécessitant pas la poignée 110. La En particulier, la circuiterie de contrôle peut basculer d'une séquence complète à une séquence partielle à l'autre en fonction de l'absence de détection de contact simultané par le système de détection ou, inversement la circuiterie de contrôle peut basculer d'une séquence partielle à une séquence complète à l'autre en fonction d'une détection de contact simultané. La bascule peut aussi s'opérer entre deux mesures nécessitant la poignée, si l'utilisateur lâche la poignée.

La circuiterie de contrôle 550 peut mettre en oeuvre, en exécutant des instructions d'un programme d'ordinateur, différentes méthodes de mesures.

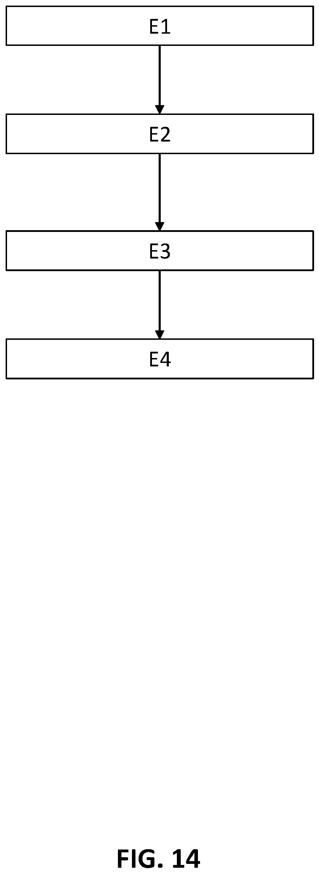

Dans une méthode d'ECG illustrée en figure 14, la circuiterie de contrôle 550 peut mettre en oeuvre les étapes suivantes :
- E1 : connexion des électrodes de contrôle 1022, 1024 au circuit de connexion électrique 1028, par le commutateur 566,
- E2 : si besoin, désactivation de la source de courant 1034 ou déconnexion de la source de courant 1034 du circuit de connexion électrique 1028,
- E3 : activation du système d'acquisition ECG 1010 pour acquérir un ECG,
- E4 : analyse et traitement de l'ECG.

L'étape E4 peut comprendre : calcul de rythme cardiaque, identification d'anomalie.

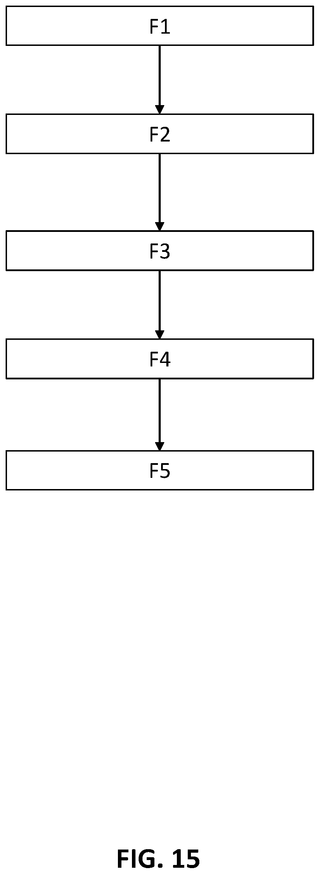

Dans une méthode d'ECG et impédancemétrie (par exemple IPG), illustrée en figure 15, la circuiterie de contrôle 550 peut mettre en oeuvre les étapes suivantes :
- F1 : connexion des électrodes de contrôle 1022, 1024 au circuit de connexion électrique 1028, par le commutateur 566,
- F2 : connexion des électrodes de mesure 1122, 1124 au voltmètre 1126 par le commutateur (les électrodes d'injection de courant 1114, 1112 étant déjà connectées à la source de courant via le circuit de connexion électrique 1026),
- F3 : activation simultanée du système d'acquisition ECG 1010 et du système d'impédancemétrie 1110 (et notamment de la source de courant de 1034 pour injecter un courant dans le corps de l'utilisateur) pour acquérir un ECG et une mesure d'impédance (par exemple un IPG),
- F4 : analyse et traitement de l'ECG et de la mesure d'impédance (par exemple IPG).

L'étape F4 peut comprendre les mêmes calculs que l'étape E4, avec peut inclure en outre la détermination d'informations relatives à la PWV (« *pulse wave velocity* », vitesse d'onde de pouls), ou la pression artérielle, ou/ou le PAT (« *pulse arrival time* », temps d'arrivée de pouls). Ces mesures supplémentaires sont permises par l'ajout d'une mesure d'impédance simultanée (un IPG dans le cas présent). L'étapes E3 et/ou l'étape F3 peuvent également inclure l'acquisition d'un BCG de façon synchronisée. La circuiterie de contrôle 550 peut contrôler l'acquisition d'un BCG, à l'aide des cellules de charge de la base 102. La station de mesure 100 permet alors d'obtenir un ECG, un IPG et un BCG qui sont synchronisés.

## Revendications

1. Station de mesure (100) comprenant :
- un système d'acquisition d'électrocardiogramme, dit système d'acquisition ECG (1010),
- deux électrodes de contrôle (1022, 1024) configurées pour être au contact d'un utilisateur,
- un circuit de connexion électrique (1026), le circuit de connexion électrique comprenant une boucle de rétroaction (1028) connectée aux deux électrodes de contrôle (1022, 1024).

2. Station de mesure (100) selon la revendication 1, dans laquelle :
- l'une des deux électrodes de contrôle (1022) est configurée pour mesurer un potentiel du corps,
- la boucle de rétroaction (1028) est configurée pour amplifier et inverser le potentiel mesuré, et
- l'autre des deux électrodes de contrôle (1024) est configurée pour mettre le corps au potentiel amplifié et inversé.

3. Station de mesure (100) selon l'une des revendications 1 à 2, dans laquelle :
- le système d'acquisition ECG (1010) comprend :
∘ une pluralité d'électrodes ECG (RA, LA, LL), configurées pour être au contact d'un utilisateur,
∘ un circuit électronique ECG (1012), connecté à la pluralité d'électrodes ECG.

4. Station de mesure selon la revendication 3, dans laquelle les deux électrodes de contrôle (1022, 1024) sont distinctes de la pluralité d'électrodes ECG (RA, LA, LL).

5. Station de mesure selon la revendication 3 ou 4, dans laquelle le circuit de connexion électrique (1026) est électriquement indépendant du circuit électronique ECG (1012).

6. Station de mesure (100) selon l'une quelconque des revendications 1 à 5, dans laquelle la boucle de rétroaction (1028) comprend :
- un amplificateur opérationnel (1030), et
- une connexion de rétroaction (1032), reliant une sortie de l'amplificateur opérationnel (1030) à une entrée de l'amplificateur opérationnel, la connexion de rétroaction comprenant au moins un composant passif (RLC),
dans laquelle par exemple le composant passif comprend une résistance et/ou un condensateur,
dans laquelle par exemple les électrodes de contrôle (1022, 1024) sont reliées respectivement à l'entrée et à la sortie de l'amplificateur opérationnel (1030).

7. Station de mesure (100) selon l'une quelconque des revendications 1 à 6, comprenant un système d'impédancemétrie (1100), le système d'impédancemétrie comprenant :
- une source de courant (1034), par exemple une source de courant alternatif,
- deux électrodes d'injection de courant (1112, 1114), pour injecter un courant dans le corps d'un utilisateur,
et dans laquelle la source de courant (1034) est reliée au circuit de connexion électrique (1026) et les deux électrodes d'injection de courant (1112, 1114) sont les deux électrodes de contrôle (1022, 1024).

8. Station de mesure (100) selon la revendication 7, comprenant un commutateur (566) permettant de déconnecter le circuit de connexion électrique (1026) et la source de courant (1034), ou dans laquelle la source de courant (1034) est désactivable.

9. Station de mesure (100) selon l'une quelconque des revendications 7 à 8, dans laquelle le système d'impédancemétrie (1100) comprend deux électrodes de mesure (1122, 1124), aptes à mesurer une différence de potentiel d'une portion du corps de l'utilisateur traversée par un courant généré par la source de courant (1034).

10. Station de mesure (100) selon l'une quelconque des revendications 7 à 9, dans laquelle le système d'acquisition d'ECG et le système d'impédancemétrie sont configurés pour être activés simultanément pour acquérir un ECG et une mesure d'impédance synchronisés, par exemple un impédance-pléthysmogramme, IPG.

11. Station de mesure selon l'une quelconque des revendications 1 à 10, comprenant une base (102) apte à recevoir les pieds d'un utilisateur et sur laquelle est montée au moins une électrode de contrôle (1022, 1024).

12. Station de mesure selon la revendication 11 en combinaison avec la revendication 3, comprenant en outre une poignée (110), apte à recevoir les mains d'un utilisateur, et sur laquelle est montée au moins une électrode parmi les électrodes ECG,
dans laquelle par exemple :
- les électrodes de contrôle (1022, 1024) sont montées sur la base (102),
- deux électrodes ECG (RA, LA) sont montées sur la poignée (110), et
- une électrode ECG (LL) est montée sur la base (102).

13. Procédé d'acquisition d'un électrocardiogramme, ECG, à l'aide d'une station de mesure (100) selon l'une quelconque des revendications 1 à 12, comprenant les étapes suivantes :
- E1 : connexion des électrodes de contrôle (1022, 1024) au circuit de connexion électrique (1026), par un commutateur (566),
- E3 : activation du système d'acquisition ECG (1010) pour acquérir un ECG.

14. Procédé selon la revendication 13, la station de mesure (100) étant selon l'une quelconque des revendications 1 à 13 en combinaison avec la revendication 7, le procédé comprenant l'étape suivante, mise en oeuvre avant l'étape E3 d'activation du système d'acquisition ECG :
- E2 : désactivation de la source de courant (1034) ou déconnexion de la source de courant (1034) du circuit de connexion électrique (1026).

15. Procédé d'acquisition d'un électrocardiogramme, ECG, et d'une mesure d'impédance à l'aide d'une station de mesure (100) selon l'une quelconque des revendications 1 à 12 en combinaison avec la revendication 7, comprenant les étapes suivantes :
- F1 : connexion des électrodes de contrôle (1022, 1024) au circuit de connexion électrique (1028), par le commutateur (566),
- F3 : activation simultanée du système d'acquisition ECG 1010 et du système d'impédancemétrie 1110 pour acquérir un ECG et une mesure d'impédance, par exemple un impédanceplethysmogramme, IPG.

16. Station de mesure (100) comprenant :
- un système d'impédancemétrie comprenant :
∘ au moins deux électrodes d'injection, pour injecter un courant dans un utilisateur, et
∘ un circuit de connexion électrique (1026), le circuit de connexion électrique comprenant une boucle de rétroaction (1028) connectée aux deux électrodes d'injection,
- un système d'acquisition d'électrocardiogramme, ECG, configuré pour acquérir des signaux d'ECG,
- une première électrode de contrôle, configurée pour modifier le potentiel du corps de l'utilisateur, la première électrode de contrôle étant l'une des électrodes d'injection de courant.

17. Station de mesure (100) selon la revendication 16, comprenant une deuxième électrode de contrôle, la deuxième électrode de contrôle étant l'autre des deux électrodes d'injection de courant.

18. Station de mesure (100) selon l'une des revendications 16 à 17, dans laquelle :
- le système d'acquisition ECG (1012) comprend :
∘ une pluralité d'électrodes ECG (RA, LA, LL), configurées pour être au contact d'un utilisateur,
∘ un circuit électronique ECG, connecté à la pluralité d'électrodes ECG, et
dans laquelle une des électrode de contrôle est une des électrodes ECG.

19. Station de mesure (100) comprenant :
une base (102) configurée pour recevoir les pieds d'un utilisateur et comprenant au moins une électrode de pied (LL) apte à être en contact avec un pied d'un utilisateur,
une poignée (110) configurée pour recevoir les mains d'un utilisateur et comprenant au moins une électrode de main (402),
un système de détection configuré pour détecter un contact simultané de l'utilisateur avec l'électrode de pied (LL) et avec l'électrode de main.

20. Station de mesure (100) selon la revendication 19, dans laquelle le système de détection est configuré pour mettre une électrode des deux électrodes (LL) à un potentiel et pour détecter par l'autre électrode (402) un potentiel supérieur à un seuil.

21. Station de mesure (100) selon l'une quelconque des revendications 19 à 20, dans laquelle le système de détection comprend :
- un générateur de tension apte à mettre ladite électrode au potentiel et
- un voltmètre apte à mesure le potentiel de ladite électrode.

22. Station de mesure (100) selon l'une quelconque des revendications 19 à 21, comprenant un système d'acquisition de mesure (BIA, ECG, IPG) utilisant les deux électrodes, et dans laquelle la station de mesure (100) est configurée pour déclencher le système d'acquisition de mesure en réponse à une détection d'un contact simultané par le système de détection.

23. Station de mesure (100) selon l'une quelconque des revendications 19 à 22, comprenant une circuiterie de contrôle (550) configurée pour mettre en oeuvre une séquence prédéterminée de mesures à exécuter par un ou plusieurs systèmes d'acquisition de mesure (BIA, ECG, IPG), et dans laquelle les mesures nécessitant la poignée (110) ne sont pas exécutées en l'absence de détection d'un contact simultané par le système de détection.

24. Station de mesure (100) selon l'une quelconque des revendications 19 à 23, dans laquelle la circuiterie de contrôle passe d'une mesure à l'autre, sans nécessiter de mesure au moyen de la poignée.

25. Station de mesure (100) selon l'une quelconque des revendications 19 à 24, comprenant une circuiterie de contrôle (550) configurée pour mettre en oeuvre une séquence prédéterminée de mesures à exécuter par un ou plusieurs systèmes d'acquisition de mesure (BIA, ECG, IPG), et dans laquelle les mesures nécessitant la poignée (110) sont exécutées en réponse à la 'détection d'un contact simultané par le système de détection.

26. Station de mesure (100) selon l'une quelconque des revendications 19 à 25, dans laquelle le système d'acquisition de mesure comprend un système d'acquisition ECG ou un système d'impédancemétrie.

27. Station de mesure (100) l'une quelconque des revendications 19 à 26, dans laquelle, en réponse à une absence de détection d'un contact simultané, le système d'acquisition ECG n'est pas activé.

28. Station de mesure selon l'une quelconque des revendications 19 à 27, comprenant une circuiterie de contrôle apte à mettre en oeuvre les étapes suivantes :
- imposition d'un potentiel à une électrode,
- mesure du potentiel au niveau de l'autre électrode,
- détermination que le potentiel mesuré est au moins égal à un seuil prédéterminé, le seuil dépendant du potentiel imposé.

29. Station de mesure selon la revendication 28, dans laquelle, en réponse à la détection d'un contact simultané, la circuiterie de contrôle permet une acquisition de mesure utilisant les électrodes de la poignée.

30. Station de mesure selon l'une quelconque des revendications 28 à 29 dans laquelle le potentiel imposé est compris entre 0,5V et 2V.

31. Station de mesure (100) selon l'une quelconque des revendications 19 à 30, dans laquelle la base (102) comprend un système de mesure de poids incluant un capteur de poids et la station de mesure est configurée pour activer le système de détection en réponse à une détection de poids par le système de mesure de poids.

32. Station de mesure (100) selon l'une quelconque des revendications 19 à 31, comprenant une circuiterie de contrôle stockant une séquence de mesures comprenant des mesures avec poignées et des mesures sans poignées et une séquence de mesure comprenant uniquement des mesures sans poignées, dans laquelle la circuiterie de contrôle est configuré pour basculer d'une séquence à l'autre en fonction de la détection par le système de détection.
